# EUROPEAN PATENT APPLICATION

(11) **EP 4 685 153 A1**
(43) Date of publication of application: **28.01.2026**
(21) Application number: 23927940.9
(22) Date of filing: 20.03.2023
(51) Int. Cl.: C07K 14/16, C07K 14/47, C07K 1/107, C12N 15/12, C12N 15/63, C12N 5/10, A61P 31/18, A61K 38/17

(54) **BROAD-SPECTRUM VIRAL MEMBRANE FUSION INHIBITOR, AND PREPARATION METHOD THEREFOR AND USE THEREOF**

(71) Applicant: Henan Genuine Biotech Co., Ltd., Pingdingshan, Henan 467000 (CN)
(72) Inventor: HE, Yuxian, Beijing 100730 (CN); ZHU, Yuanmei, Beijing 100730 (CN); CHONG, Huihui, Beijing 100730 (CN); GENG, Xiuzhu, Beijing 100730 (CN)
(74) Representative: Botti & Ferrari S.p.A.
(86) International application number: PCT/CN2023/082491
(87) International publication number: WO 2024/192623

(57) **Abstract**

The present disclosure relates to a lipopeptide or a pharmaceutically acceptable salt, solvate, hydrate, complex, chelate, non-covalent complex or a prodrug thereof, which is a broad-spectrum viral membrane fusion inhibitor, can strongly inhibit a human immunodeficiency virus and multiple other viruses, and can be used for treating a human immunodeficiency virus infection, multiple other virus infections, and diseases caused by the infections.

## Description

### Technical Field

The present disclosure belongs to the biomedicine field, and it relates to a lipopeptide (or a pharmaceutically acceptable salt, a solvate, a hydrate, a complex, a chelate, a non-covalent complex or a prodrug thereof), and further relates to a polypeptide (or a variant thereof). The lipopeptide (or a pharmaceutically acceptable salt, a solvate, a hydrate, a complex, a chelate, a non-covalent complex or a prodrug thereof) being a broad-spectrum viral membrane fusion inhibitor which can potently inhibit a human immunodeficiency virus and multiple other viruses, and thus can be used for treating a human immunodeficiency virus infection, multiple other virus infections, and diseases caused by the infections.

### Background Art

Infections caused by viruses, such as Human Immunodeficiency Virus (HIV), Novel Coronavirus (SARS-CoV-2), Middle East Respiratory Syndrome Virus (MERS-CoV), Ebola virus (EBOV), Marburg Virus (MARV), Hepatitis B Virus (HBV), Influenza Virus (IAV), Rabies Virus (RABV), Vesicular Stomatitis Virus (VSV), and Respiratory Syncytial Virus (RSV), severely endanger human health and social stability. Effective antiviral drugs, especially drugs with broad-spectrum antiviral effect, are in a great global shortage. Membrane fusion, such as formation of fertilized eggs and transport and release of intracellular substances, is an extremely important biological phenomenon. Many viruses, including the above mentioned viruses, infect host cells through the pathway of membrane fusion. Developing specific or broad-spectrum antiviral drugs targeting the common mechanisms of viral membrane fusion holds significant scientific and practical value.

HIV infection leads to Acquired Immune Deficiency Syndrome (AIDS). At present, neither effective vaccines to prevent HIV nor drugs for entirely curing the HIV infection exist. Existing anti-HIV drugs include reverse transcriptase inhibitors, protease inhibitors, viral entry inhibitors, integrase inhibitors and the like, and they work by blocking the replication of the virus at different stages and play a major role in the treatment and prevention of AIDS. The widely used highly active antiretroviral therapy ("cocktail" therapy) consists mainly of three to four reverse transcriptase inhibitors and protease inhibitors. However, due to the persistence of HIV infection, it is required to administer drugs to patients for a long period of time, easily leading to drug resistance, which seriously affects the clinical treatment effect. Accordingly, the development of new anti-HIV drugs has always been an important demand for preventing and controlling AIDS.

Entry of HIV into host cells is mediated by the trimeric envelope glycoprotein (Env) on its surface, which includes a surface subunit gp120 and a transmembrane subunit gp41. During HIV infection process, gp120 first binds to the receptor CD4 and then to the co-receptor (such as CCR5 or CXCR4), which induces conformational changes in gp120, thereby exposing gp41 and activating the membrane fusion function of the gp41. The N-terminal fusion peptide (FP) of gp41 is first inserted into the membrane of a target cell, then the C-terminal heptad region (CHR) is reversely bound to the N-terminal heptad region (NHR) to form a stable six-helix bundle (6-HB) structure, thereby bringing the viral membrane close to the cell membrane for fusion, to make the HIV genetic material enter into the cell to result in infections. The crystalline structure^{[1]} shows that the C-terminus of the NHR helix contains a hydrophobic deep pocket, which has been always recognized as an important antiviral drug target, while the sequence "WMEWDREI" at the N-terminus of the CHR helix is a NHR pocket-binding domain (PBD), where the eight amino acids are located at the positions *a, b, c, d, e, f, g* and *a* of the CHR helix, respectively. Among them, the two W (tryptophan) at the positions *a* and *d* and the I (isoleucine) at the position *a* are hydrophobic amino acids, and they are inserted into the NHR pocket to mediate extensive hydrophobic effects, which is crucial for the formation and function of the viral fusion protein 6-HB.

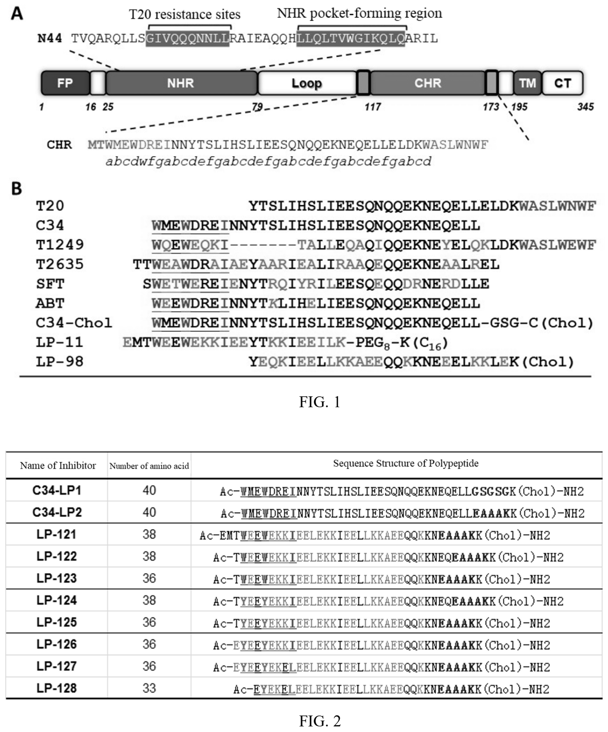

Viral membrane fusion inhibitors act at the early stages of virus replication by blocking the virus from entering the target cells, and thus they have obvious advantages in both treatment and prevention. However, only one membrane fusion inhibitor currently has been approved by U.S Food and Drug Administration (FDA) for clinical application, which is the HIV membrane fusion inhibitor enfuvirtide (also known as T20). As shown in FIG. 1, the T20 is a polypeptide derived from the gp41 CHR and free of the PBD sequence, consisting of 36 amino acids, and it exerts its antiviral effect by competitively binding to the NHR to block the formation of the 6-HB of the virus. The T20, due to its relatively low activity for inhibiting virus and its relatively short half-life, is required to be administered at a large dose every day (2 times per day, 90 mg for each time). T20 easily induces drug resistance, thereby making the clinical treatment in failure. Hence, the development of new HIV membrane fusion inhibitors is always concerned at home and abroad.

At present, the research and development of HIV membrane fusion inhibitors are mostly focused on the role of PBD, especially on using the C34 polypeptide published in the early years as a design template^{[2]}. The C34 contains PBD and its amino acid sequence is recognized as the core sequence of the CHR. Following the T20, original pharmaceutical companes have further designed a second-generation inhibitor T1249 and a third-generation inhibitor T2635^{[3]}, both of which contain the PBD sequence (see FIG. 1). Both Sifuvirtide (SFT) and Albuvirtide (ABT), which are early developed in China, are designed based on the C34 sequence^{[4][5]}. The SFT is obtained by mutating 14 amino acids of the C34, and adding serine (S) and glutamic acid (E) at the N-terminus and the C-terminus, respectively, to increase the stability and target binding ability of the polypeptide. The ABT is obtained by only mutating three amino acids of the C34, and incorporating 3-maleimidopropanoic acid (MPA) to the lysine (K) side chain at position 13 to make it has the ability for binding serum albumin. Although the antiviral activities of both the SFT and the ABT are not substantially improved in comparison to the T20 and the C34, they have prolonged biological half-life so that the administration frequency may be reduced. Modifying the C-terminus of the C34 polypeptide with cholesterol molecules to form a C34-Chol lipopeptide can significantly improve antiviral activity and biological half life^{[6]}.

The present inventor team has long been engaged in research and development of HIV membrane fusion inhibitors, and has designed multiple CHR polypeptide-based HIV membrane fusion inhibitors according to the structure and function relationship of the gp41^{[7]}. Among them, LP-11 contains a PBD motif, and its C-terminus is modified with palmitic acid molecules; while LP-98 does not contain the PBD motif, and its C-terminus is modified with cholesterol. In comparison to the LP-11, the LP-98 has significantly improved antiviral activity. However, the inventors also find that the LP-98 has a significantly reduced inhibitory activity against T20 resistant HIV strains so that its druggability is affected. Therefore, there is a need in the art for persistent research and development of a novel viral membrane fusion inhibitor medicament, to satisfy clinical needs.

The diagram for sequence structures of T20, C34, T1249, T2635, SFT, ABT, C34-Chol, LP-11 and LP-98 mentioned above is shown in FIG. 1.

### Summary

The technical problem to be solved by the present disclosure is how to effectively inhibit HIV and some other viruses with membrane fusion function. The inventors of the present disclosure are devoted to developing a potent, broad-spectrum and long-acting viral membrane fusion inhibitor for a long time. In the research, the inventors surprisingly discover that based on the CHR sequence of HIV, the introduction of a "rigid linker" and the substitution of some amino acids in the PBD can significantly enhance the antiviral activity of the polypeptide.

Hence, in one aspect, the present disclosure provides a lipopeptide or a pharmaceutically acceptable salt thereof, the lipopeptide being a viral membrane fusion inhibitor can inhibit viruses such as HIV, SARS-CoV-2 and mutant strains thereof, MERS-CoV, EBOV, MARV, IAV, RABV, and VSV.

In another aspect, the present disclosure provides a polypeptide or a variant thereof, and a conjugate comprising the polypeptide or the variant thereof.

In another aspect, the disclosure provides an isolated nucleic acid encoding the polypeptide or the variant thereof, a vector comprising the isolated nucleic acid, and a host cell comprising the isolated nucleic acid or the vector.

In another aspect, the disclosure provides a multimer formed from the lipopeptide or the pharmaceutically acceptable salt thereof or the polypeptide or the variant thereof.

In another aspect, the present disclosure provides a composition comprising the lipopeptide or the pharmaceutically acceptable salt thereof, the polypeptide or the variant thereof, the conjugate, the isolated nucleic acid, the vector, the host cell or the multimer, and a pharmaceutical composition comprising the lipopeptide or the pharmaceutically acceptable salt thereof or the polypeptide or the variant thereof.

In another aspect, the present disclosure provides use of the lipopeptide or the pharmaceutically acceptable salt thereof or the polypeptide or the variant thereof in the manufacture of a medicament or in the treatment of a disease.

### Detailed Description of the Invention

The purposes and implementations of the present disclosure will be further described in detail below. In the technique of designing a lipopeptide-based viral membrane fusion inhibitor, it is typically necessary to add a linker between a polypeptide sequence and an aliphatic group (e.g., fatty acids and cholesterol, etc.) to act as a linker arm. The expected binding site of the aliphatic groups is a virus membrane or a cell membrane which makes the polypeptide enriched in a target region, thereby significantly improving the antiviral activity of the polypeptide. Because a polypeptide tends to form a stable secondary structure and has strong structural rigidity, a flexible linker is typically selected for the connection between the polypeptide and the aliphatic group, so that the polypeptide and the aliphatic group can form suitable conformations respectively and bind to their respective binding sites, and their respective effects are sufficiently exerted while avoiding the interaction caused by steric hindrance and other reasons. Common flexible linkers include a combination of glycine (G) and serine (S), e.g., (GGGGS)n or (GSGSG)n, where the size of n can be adjusted to increase or decrease the distance between domains. Another common flexible linker is a small molecule polyethylene glycol (PEG)n, where n is mostly between 2 and 24. At present, all viral membrane fusion inhibitor lipopeptides reported in literatures use a flexible linker. For example, the HIV membrane fusion inhibitor C34-Chol uses GSG, and the LP-11 uses PEG₈; for another example, the SARS-CoV-2 membrane fusion inhibitor IPB02V3 uses PEG₈, the IPB24 to IPB27 use respectively PEG₄, PEG₅, PEG₆ and PEG₈^{[8]}, the EKL1C uses GSG^{[9]}, the EK1C4 uses a tandem GSGSG and PEG₄^{[8]}, [SARS_{HRC}-PEG₄]₂-chol uses PEG₄^{[10]}, etc.

(EAAAK)n sequence capable of forming an alpha-helix is a common rigid linker for preparing fusion proteins, which has an internal hydrogen bond and closely contacts with the backbone of the peptide chain, and is rigid and stable, thereby effectively separating the domains. However, there is no precedent for currently prepared lipopeptide HIV membrane fusion inhibitors to use a rigid linker. The present disclosure prepares a lipopeptide by using a rigid linker EAAAK sequence, and it is surprisingly found that the rigid linker confers a significant helical structure and potent antiviral activity to the polypeptide. In a specific example of the present disclosure, with a C34 polypeptide as the design template, lipopeptides C34-LP1 and C34-LP2 were first prepared through a GSGSG flexible linker and a EAAAK rigid linker, respectively, and the results show that the EAAAK rigid linker helps to improve the anti-HIV activity of the lipopeptide. Further, three novel lipopeptides LP-121, LP-122 and LP-123 using EAAAK as a linker are designed, and at the corresponding positions b, c and f, g of the CHR helix, EE and KK amino acid pairs are introduced, to promote the formation of a "salt bridge" structure at the i and i+4 positions. It is unexpectedly found with experiments that the LP-123 lipopeptide containing a shorter sequence has optimal anti-HIV activity.

The amino acid WMEWDREI at the N-terminus of the CHR helix in gp41 is a pocket-binding domain (PBD), wherein the two W at the positions *a* and *b* of the CHR helix and the I at the position *a* are inserted into the NHR pocket to mediate extensive hydrophobic effects, which plays an important role in the formation of the fusion protein 6-HB. Hence, the research and development of new generation HIV membrane fusion inhibitors are mostly focused on the effects of PBD, especially on using a PBD-containing C34 polypeptide as the design template. Representative inhibitors in this field include T1249, T2635, SFT, ABT, and C34-Chol, etc. While amino acid substitution is a common strategy for optimizing polypeptide inhibitors, for HIV membrane fusion inhibitors as currently reported in literatures, the two W in the PBD sequence are used without exception, and there is no precedent to substitute the W with other amino acids. To further improve the antiviral activity of inhibitors, it is surprisingly found in the present disclosure that the substitution of the two hydrophobic W in the PBD with two hydrophilic and small molecular weight tyrosine (Y) can significantly improve the antiviral activity of HIV membrane fusion inhibitors. In the specific implementation process, LP-124 and LP-125 are prepared by using the above LP-122 and LP-123 lipopeptide as a template respectively, and with experiments, it is verified that the substitution of W with Y can remarkably improve the antiviral activity of the inhibitors. Such a technical strategy changes the interaction between the PBD and the NHR pocket from hydrophobic interaction to hydrophilic interaction, offering a new approach for the design of HIV membrane fusion inhibitors in the field.

Further, the present disclosure, based on LP-125, prepares the lipopeptide LP-126 by replacing the first amino acid T at the N-terminus with a negatively charged amino acid E, to further enhance the hydrophilic interaction and stability of the lipopeptide N-terminus. More further, the lipopeptide LP-127 is prepared by reducing the amino acid at the 8^{th} position of the LP-126 to E and substituting the I at the position *a* with the amino acid L (leucine) having a relatively low hydrophilicity; and the LP-128 containing only one Y is prepared by removing three amino acids at the N-terminus of the LP-127. These novel inhibitors share the common characteristics of containing an EAAAK linker, a PBD motif with W replaced by Y, and rich EE-KK salt bridge-forming amino acids. In terms of the amino acid sequence, only 8 amino acids retain the amino acids of the original CHR sequence. Through specific experiments, it is found that these novel inhibitors have potent anti-HIV activity. More unexpectedly, it is found that the LP-127 and the LP-128 can effectively inhibit infections of pseudoviruses such as SARS-CoV-2, MERS-CoV, EBOV, MARV, IAV, and RABV, reflecting their broad-spectrum antiviral activity.

The diagram for sequence structures of the above LP-121, LP-122, LP-123, LP-124, LP-125, LP-126, LP-127 and LP-128 is shown in FIG. 2. In the structural formulas of the lipopeptide shown in FIG. 2, Ac represents an acetyl group, which is an amino-terminal protecting group of the polypeptide, and Chol represents a cholesterol molecule for modifying the C-terminus of the polypeptide, and the modification is on the side chain of the amino acid K at the C-terminus of the polypeptide. NH₂ represents an amino group, which is a carboxyl-terminal protecting group of the polypeptide.

The present disclosure is at least partially based on the above-described unexpected discovery of the inventors. Thus, in one aspect, the present disclosure provides a lipopeptide or a pharmaceutically acceptable salt, a solvate, a hydrate, a complex, a chelate, a non-covalent complex or a prodrug thereof, comprising a polypeptide or a variant thereof and a modification group linked to the C-terminus of the polypeptide or the variant thereof through a linker, and optionally a terminal protecting group of the polypeptide,

(X₁X₂X₃)mX₄X₅X₆X₇X₈X₉X₁₀X₁₁X₁₂X₁₃X₁₄X₁₅IX₁₆X₁₇LX₁₈X₁₉X₂₀X₂₁X₂₂X₂₃QQX₂₄X₂₅N(EX₂₆)n Formula I

in which, the polypeptide is represented by Formula I; the modification group is a lipophilic compound; the linker is -(EAAAK)n₁-, -(XP)n₂-, -(EAAAK)n₁-X₂₇- or -(XP)n₂-X₂₇-,
X₁ is T, E or S; X₂ is W or a hydrophilic amino acid; X₃ is E, M or Q; m is 0 or 1; X₄ is E, A or T; X₅ is W or a hydrophilic amino acid; X₆ is E or D, X₇ is R, K or Q; X₈ is E, K or A; X₉ is L or I; X₁₀ is E, N or A; X₁₁ is E or N; X₁₂ is L or Y; X₁₃ is E, T or A; X₁₄ is K, S, R or A; X₁₅ is K, L, R or Q; X₁₆ is E, H, T or Y; X₁₇ is E, S, R or A; X₁₈ is L or I; X₁₉ is K, E or R; X₂₀ is K, E, Q or A; X₂₁ is A or S; X₂₂ is E or Q; X₂₃ is E, N or I; X₂₄ is K, E or D; X₂₅ is K or R; X₂₆ is Q, E, R, A or Y; n is 0 or 1;
X is any one amino acid; X₂₇ is K or C; n₁ is a natural number between 1 and 5; n₂ is a natural number between 1 and 5,
and the variant differs from the polypeptide from which the variant is derived only in the substitution (e.g., a conservative substitution or non-conservative substitution) of one or several (such as 1, 2, 3, 4 or 5) amino acid residues, and retains the biological function of the polypeptide from which the variant is derived.

In some embodiments, X₁ is T or E. In some embodiments, X₁ is T. In some embodiments, X₁ is E.

In some embodiments, X₂ is W, D, E, H, K, Q, R, S, T or Y. In some embodiments, X₂ is W or Y. In some embodiments, X₂ is Y. In some embodiments, X₂ is W.

In some embodiments, X₃ is E or M. In some embodiments, X₃ is E. In some embodiments, X₃ is M.

In some embodiments, X₄ is E.

In some embodiments, X₅ is W, D, E, H, K, Q, R, S, T or Y. In some embodiments, X₅ is W or Y. In some embodiments, X₅ is Y. In some embodiments, X₅ is W.

In some embodiments, X₆ is E.

In some embodiments, X₇ is R or K. In some embodiments, X₇ is R. In some embodiments, X₅ is K.

In some embodiments, X₈ is E or K. In some embodiments, X₈ is E. In some embodiments, X₈ is K.

In some embodiments, X₁₀ is E or N. In some embodiments, X₁₀ is E. In some embodiments, X₁₀ is N.

In some embodiments, X₁₁ is E.

In some embodiments, X₁₂ is L.

In some embodiments, X₁₃ is E or T. In some embodiments, X₁₃ is E. In some embodiments, X₁₃ is T.

In some embodiments, X₁₄ is K or S. In some embodiments, X₁₄ is K. In some embodiments, X₁₄ is S.

In some embodiments, X₁₅ is K or L. In some embodiments, X₁₅ is K. In some embodiments, X₁₅ is L.

In some embodiments, X₁₆ is E or H. In some embodiments, X₁₆ is E. In some embodiments, X₁₆ is H.

In some embodiments, X₁₇ is E or S. In some embodiments, X₁₇ is E. In some embodiments, X₁₇ is S.

In some embodiments, X₁₈ is L.

In some embodiments, X₁₉ is K or E. In some embodiments, X₁₉ is K. In some embodiments, X₁₉ is E.

In some embodiments, X₂₀ is K or E. In some embodiments, X₂₀ is K. In some embodiments, X₂₀ is E.

In some embodiments, X₂₁ is A.

In some embodiments, X₂₂ is E.

In some embodiments, X₂₃ is E or N. In some embodiments, X₂₃ is E. In some embodiments, X₂₃ is N.

In some embodiments, X₂₄ is K or E. In some embodiments, X₂₄ is K. In some embodiments, X₂₄ is E.

In some embodiments, X₂₅ is K.

In some embodiments, X₂₆ is Q.

In some embodiments, X is A, K or E.

In some embodiments, m is 0. In some embodiments, m is 1.

In some embodiments, n is 0. In some embodiments, n is 1.

In some embodiments, X₂₇ is K. In some embodiments, X₂₇ is C.

In some embodiments, n₁ is a natural number between 1 and 4. In some embodiments, n₁ is a natural number between 1 and 3. In some embodiments, n₁ is 1 or 2. In some embodiments, n₁ is 1. In some embodiments, n₁ is 2. In some embodiments, n₁ is 3. In some embodiments, n₁ is 4. In some embodiments, n₁ is 5.

In some embodiments, n₂ is a natural number between 1 and 4. In some embodiments, n₂ is a natural number between 1 and 3. In some embodiments, n₂ is 1 or 2. In some embodiments, n₂ is 1. In some embodiments, n2 is 2. In some embodiments, n₂ is 3. In some embodiments, n₂ is 4. In some embodiments, n₂ is 5.

In some embodiments, X₂₅ is K, and the polypeptide is represented by Formula II,

(X₁X₂X₃)mX₄X₅X₆X₇X₈X₉X₁₀X₁₁X₁₂X₁₃X₁₄X₁₅IX₁₆X₁₇LX₁₈X₁₉X₂₀X₂₁X₂₂X₂₃QQX₂₄KN(EX₂₆)n Formula II

in which, the definitions of X₁, X₂, X₃, m, X₄, X₅, X₆, X₇, X₈, X₉, X₁₀, X₁₁, X₁₂, X₁₃, X₁₄, X₁₅, X₁₆, X₁₇, X₁₈, X₁₉, X₂₀, X₂₁, X₂₂, X₂₃, X₂₄, X₂₆, and n are as described in any embodiment according to the present disclosure.

In some embodiments, X₄ is E, X₂₅ is K, and the polypeptide is represented by Formula III,

(X₁X₂X₃)m-EX₅X₆X₇X₈X₉X₁₀X₁₁X₁₂X₁₃X₁₄X₁₅IX₁₆X₁₇LX₁₈X₁₉X₂₀X₂₁X₂₂X₂₃QQX₂₄KN(EX₂₆)n Formula III

in which, the definitions of X₁, X₂, X₃, m, X₅, X₆, X₇, X₈, X₉, X₁₀, X₁₁, X₁₂, X₁₃, X₁₄, X₁₅, X₁₆, X₁₇, X₁₈, X₁₉, X₂₀, X₂₁, X₂₂, X₂₃, X₂₄, X₂₆, and n are as described in any embodiment according to the present disclosure.

In some embodiments, X₄ is E, X₁₀ is E, X₁₁ is E, X₁₂ is L, X₁₃ is E, X₁₄ is K, X₁₅ is K, X₁₆ is E, X₁₇ is E, X₁₈ is L, X₁₉ is K, X₂₀ is K, X₂₁ is A, X₂₂ is E, X₂₃ is E, X₂₄ is K, X₂₅ is K, and the polypeptide is represented by Formula IV,

(X₁X₂X₃)m-EX₅X₆X₇X₈X₉EELEKKIEELLKKAEEQQKKN(EX₂₆)n Formula IV

In which, the definitions of X₁, X₂, X₃, m, X₅, X₆, X₇, X₈, X₉, X₂₆, and n are as described in any embodiment according to the present disclosure.

In some embodiments, X₄ is E, X₆ is E, X₇ is K, X₁₀ is E, X₁₁ is E, X₁₂ is L, X₁₃ is E, X₁₄ is K, X₁₅ is K, X₁₆ is E, X₁₇ is E, X₁₈ is L, X₁₉ is K, X₂₀ is K, X₂₁ is A, X₂₂ is E, X₂₃ is E, X₂₄ is K, X₂₅ is K, and the polypeptide is represented by Formula V,

(X₁X₂X₃)m-EX₅EKX₈X₉EELEKKIEELLKKAEEQQKKN(EX₂₆)n Formula V

In which, the definitions of X₁, X₂, X₃, m, X₅, X₈, X₉, X₂₆, and n are as described in any embodiment according to the present disclosure.

In some embodiments, the terminal protecting group of the polypeptide comprises an N-terminal (amino-terminal) protecting group and/or a C-terminal (carboxy-terminal) protecting group. In some embodiments, the lipopeptide according to the present disclosure comprises a N-terminal protecting group and a C-terminal protecting group. As well known, the N-terminal protecting group may be any one selected from the group consisting of acetyl (Ac), amino (NH₂), maleoyl, succinyl, tert-butoxycarbonyl, benzyloxy, another hydrophobic group and macromolecular carrier group. As well known, the C-terminal protecting group may be any one selected from the group consisting of amino (NH₂), carboxyl, hydroxyl, amido, tert-butoxycarbonyl, another hydrophobic and macromolecular carrier group. In some embodiments, the N-terminal protecting group is acetyl (Ac). In some embodiments, the C-terminal protecting group is amino (NH₂).

The abbreviations of amino acids in the polypeptide according to the present disclosure have the meanings well known in the art, for example: W is tryptophan, M is methionine, E is glutamic acid, D is aspartic acid, R is arginine, I is isoleucine, N is asparagine, Y is tyrosine, T is threonine, S is serine, L is leucine, H is histidine, Q is glutamine, K is lysine, A is alanine, G is glycine, and the like. In some embodiments, the amino acids are L-configured amino acids, and one or more (e.g., 2-5, 2-4, or 2-3) amino acid residues in the polypeptide may also be replaced by amino acids with chemically similar properties, such as L-configured amino acid(s), D-configured amino acid(s), artificially modified amino acid(s), or rare amino acid(s) present in nature, to improve the bioavailability, stability, and/or antiviral activity of the polypeptide, wherein the D-configured amino acid refers to an amino acid corresponding to a L-configured amino acid constituting a protein; the artificially modified amino acid refers to a common L-configured amino acid which constitutes a protein and is modified by means of methylation, phosphorylation or the like; and the rare amino acid present in nature includes an uncommon amino acids constituting a protein and an amino acid not constituting a protein, for example, 5-hydroxylysine, methylhistidine, gamma-aminobutyric acid, and homoserine.

In some embodiments, the lipophilic compound is cholesterol (Chol), fatty acid, dihydrosphingosine (DHS) or Vitamin E (tocopherol, Toc), etc. In some embodiments, the cholesterol includes: cholesteryl hemisuccinate, 2-cholesteryl acetic acid, 2-cholesteryl propionic acid, 3-cholesteryl propionic acid, 2-cholesteryl butyric acid, 2-cholesteryl isobutyric acid, 3-cholesteryl butyric acid, 3-cholesteryl isobutyric acid, 4-cholesteryl butyric acid, 2-cholesteryl valeric acid, 2-cholesteryl isovaleric acid, 3-cholesteryl valeric acid, 5-cholesteryl valeric acid, 2-cholesteryl caproic acid, 6-cholesteryl caproic acid, 2-cholesteryl enanthic acid, 7-cholesteryl enanthic acid, 2-cholesteryl caprylic acid, 8-cholesteryl caprylic acid, cholesteryl bromoacetate, etc. In some embodiments, the fatty acid includes a fatty acid containing 8 to 20 carbon atoms, e.g., octadecanoic acid or palmitic acid.

In some embodiments, the lipophilic compound is cholesterol (Chol). In some embodiments, the lipophilic compound is fatty acid. In some embodiments, the lipophilic compound is dihydrosphingosine (DHS). In some embodiments, the lipophilic compound is Vitamin E (tocopherol, Toc). In some embodiments, the cholesterol is cholesteryl hemisuccinate. In some embodiments, the lipophilic compound is 2-cholesteryl acetic acid. In some embodiments, the lipophilic compound is 2-cholesteryl propionic acid. In some embodiments, the lipophilic compound is 3-cholesteryl propionic acid. In some embodiments, the lipophilic compound is 2-cholesteryl butyric acid. In some embodiments, the lipophilic compound is 2-cholesteryl isobutyric acid. In some embodiments, the lipophilic compound is 3-cholesteryl butyric acid. In some embodiments, the lipophilic compound is 3-cholesteryl isobutyric acid. In some embodiments, the lipophilic compound is 4-cholesteryl butyric acid. In some embodiments, the lipophilic compound is 2-cholesteryl valeric acid. In some embodiments, the lipophilic compound is 2-cholesteryl isovaleric acid. In some embodiments, the lipophilic compound is 3-cholesteryl valeric acid. In some embodiments, the lipophilic compound is 5-cholesteryl valeric acid. In some embodiments, the lipophilic compound is 2-cholesteryl caproic acid. In some embodiments, the lipophilic compound is 6-cholesteryl caproic acid. In some embodiments, the lipophilic compound is 2-cholesteryl enanthic acid. In some embodiments, the lipophilic compound is 7-cholesteryl enanthic acid. In some embodiments, the lipophilic compound is 2-cholesteryl caprylic acid. In some embodiments, the lipophilic compound is 8-cholesteryl caprylic acid. In some embodiments, the lipophilic compound is cholesteryl bromoacetate. In some embodiments, the fatty acid is a fatty acid containing 8 to 20 carbon atoms. In some embodiments, the lipophilic compound is octadecanoic acid. In some embodiments, the lipophilic compound is palmitic acid.

In some embodiments, the linker is -(EAAAK)n₁-, wherein the definition of n₁ is as described in any embodiment according to the present disclosure. In some embodiments, the linker is -(XP)n₂-, wherein the definitions of X and n₂ are as described in any embodiment according to the present disclosure. In some embodiments, the linker is -(EAAAK)n₁-X₂₇-, wherein the definitions of n₂ and X₂₇ are as described in any embodiment according to the present disclosure. In some embodiments, the linker is -(XP)n₂-X₂₇-, wherein the definitions of X, n₂ and X₂₇ are as described in any embodiment according to the present disclosure.

The lipophilic compound may be either linked to the side chain of the terminal amino acid of the linker or directly linked to the linker. Among them, the fatty acid, the dihydrosphingosine and the vitamin E can achieve modification to the polypeptide by performing an amidation reaction with the side chain amino group of the lysine (Lys) in the linker; the cholesterol can achieve modification to the polypeptide either by performing an amidation reaction with the side chain amino group of the lysine (Lys) in the linker or by performing a thioether-forming reaction with the side chain sulfhydryl group of the cysteine (Cys) in the linker. In some specific embodiments, the cholesterol is cholesteryl hemisuccinate, which achieves modification to the polypeptide by performing an amidation reaction with the side chain amino group of the K in the linker. It is well known in the art that cholesteryl bromoacetate, as a modification group of the polypeptide, can also achieve modification to the polypeptide by performing a thioether-forming reaction with the side chain sulfhydryl group of the cysteine (C) in the linker.

In some embodiments, the lipopeptide has an amino acid sequence selected from amino acid sequences as set forth in SEQ ID NO: 11-19.

As well known in the art, the chemical modification of a polypeptide by use of lipids, to obtain the so-called "lipopeptides", can increase the ability to target cell membrane and antiviral activity of the polypeptide, and meanwhile, further significantly improve the stability and biological half-life of the polypeptide. Hence, as well in the art, the polypeptide according to the present disclosure also has similar antiviral activity to the lipopeptide. On this basis, in another aspect, the present disclosure further provides a polypeptide or a variant thereof, which is the polypeptide or the variant thereof as described in any of the above-described embodiments according to the present disclosure. Specifically, the polypeptide comprises or consisting of a sequence represented by Formula I, Formula II, Formula III, Formula IV or Formula V:

(X₁X₂X₃)mX₄X₅X₆X₇X₈X₉X₁₀X₁₁X₁₂X₁₃X₁₄X₁₅IX₁₆X₁₇LX₁₈X₁₉X₂₀X₂₁X₂₂X₂₃QQX₂₄X₂₃N(EX₂₆)n Formula I

(X₁X₂X₃)mX₄X₅X₆X₇X₈X₉X₁₀X₁₁X₁₂X₁₃X₁₄X₁₅IX₁₆X₁₇LX₁₈X₁₉X₂₀X₂₁X₂₂X₂₃QQX₂₄KN(EX₂₆)n Formula II

(X₁X₂X₃)m-EX₅X₆X₇X₈X₉X₁₀X₁₁X₁₂X₁₃X₁₄X₁₅IX₁₆X₁₇LX₁₈X₁₉X₂₀X₂₁X₂₂X₂₃QQX₂₄KN(EX₂₆)n Formula III

(X₁X₂X₃)m-EX₅X₆X₇X₈X₉EELEKKIEELLKKAEEQQKKN(EX₂₆)n Formula IV

(X₁X₂X₃)m-EX₅EKX₈X₉EELEKKIEELLKKAEEQQKKN(EX₂₆)n Formula V

in which, the definitions of X₁, X₂, X₃, m, X₄, X₅, X₆, X₇, X₈, X₉, X₁₀, X₁₁, X₁₂, X₁₃, X₁₄, X₁₅, X₁₆, X₁₇, X₁₈, X₁₉, X₂₀, X₂₁, X₂₂, X₂₃, X₂₄, X₂₅, X₂₆, and n are as described in any embodiment according to the present disclosure.

In some embodiments, the lipopeptide has an amino acid sequence selected from amino acid sequences as set forth in SEQ ID NO: 21-29.

As well known in the art, the amino acids corresponding to the positions *a* and *d* of CHR helix are key amino acids for the formation of the 6-HB structure by binding the CHR with the NHR target sequence, and they are conservative in sequence and function so that they are not readily substituted by other amino acids. In contrast, the amino acids corresponding to the positions *b, c, f* and g of the CHR helix have no or little direct interaction with the NHR and are therefore readily substituted by other amino acids without affecting or with only slightly affecting the antiviral activity of the polypeptide. Thus, the amino acids in the polypeptide may undergo substitution, addition, or deletion of one or more other amino acids, and the polypeptide still has the activity of inhibiting HIV and/or other viruses. The substitution of amino acids refers to the substitution of an amino acid residue at a position in the polypeptide sequence with another amino acid, preferably with a conservative amino acid, i.e., a conservative substitution; the addition of amino acids refers to the insertion of additional amino acid residue(s) at the N-terminus or C-terminus or at other appropriate position of the polypeptide sequence, and the inserted amino acid residues may be fully or partially contiguous, or non-contiguous to each other; the deletion of amino acids refers to the removal of one or more amino acid residues from the polypeptide sequence, provided that the modified polypeptide has the activity for inhibiting viruses.

The so-called "conservative amino acid" or "conservation of amino acids" has the meanings well known in the art. For example, amino acids are classified into acidic amino acids, basic amino acids and neutral amino acids according to the numbers of amino groups and carboxyl groups contained in the amino acid molecules, wherein the acidic amino acids refer to E and D, the basic amino acids refer to K, R and H (histidine), and the neutral amino acids refer to A, L, I, V, C, Y, G, M, S, T, F (phenylalanine), W and P (proline). For another example, amino acids are classified into hydrophilic amino acids (D, E, H, K, Q, R, S, T, Y) and hydrophobic amino acids (A, F, I, L, M, P, V, W) according to the hydrophilicity and the hydrophobicity. For another example, hydrophilic uncharged amino acids refer to N, Q, S and T; aliphatic uncharged amino acids refer to A, L, I, V and G; nonpolar uncharged amino acids refer to C, M and P; aromatic amino acids refer to Y, F and W. For another example, amino acids containing alcohol group include S and T; aliphatic amino acids include L, I, V and M; cycloalkenyl-related amino acids include F, H, W and Y. These amino acids with similar sizes, shapes, charges, and chemical properties including the capability of forming covalent bond or hydrogen bond, are generally considered as conservative amino acids.

In the present disclosure, the term "conservative substitution" refers to amino acid substitutions which would not disadvantageously affect or change the essential properties of a protein/polypeptide comprising the amino acid sequence. For example, a conservative substitution may be introduced by standard techniques known in the art such as site-directed mutagenesis and PCR-mediated mutagenesis. Substitutions of conservative amino acid include substitutions wherein an amino acid residue is substituted with another amino acid residue having a similar side chain, for example, a residue physically or functionally similar (such as, having similar size, shape, charge, chemical property including the capability of forming covalent bond or hydrogen bond, etc.) to the corresponding amino acid residue. The families of amino acid residues having similar side chains have been defined in the art. These families include amino acids having basic side chains (for example, lysine, arginine and histidine), amino acids having acidic side chains (for example, aspartic acid and glutamic acid), amino acids having uncharged polar side chains (for example, glycine, asparagine, glutamine, serine, threonine, tyrosine, cysteine, and tryptophan), amino acids having nonpolar side chains (for example, alanine, valine, leucine, isoleucine, proline, phenylalanine, and methionine), amino acids having β-branched side chains (such as threonine, valine, and isoleucine) and amino acids having aromatic side chains (for example, tyrosine, phenylalanine, tryptophan, and histidine). Therefore, generally a conservative substitution refers to a substitution of a corresponding amino acid residue with another amino acid residue from the same side-chain family. Methods for identifying the conservative substitutions of amino acids are well known in the art.

The present inventor team has demonstrated with more than ten years of research experiences that polypeptides derived from substitutions, additions or deletions of amino acids in a parent polypeptide with a sequence identity of up to about 30%, 40%, 50%, 60%, 70%, 80% or 90% as inhibitors, may still have potent antiviral activity. For example, for the above-described lipopeptides LP-126, LP-127, and LP-128, only 8 amino acids of the original CHR prototype are retained, and the LP-126 and LP-127 only have a sequence identity of 26.7% to the amino acids of the original CHR prototype, and the LP-128 has a sequence identity of 29.6% to the amino acids of the original CHR prototype. Thus, any one of the polypeptides is a polypeptide with a sequence identity of about 27%, 30%, 35%, 40%, 45%, 50%, 55%, 60%, 65%, 70%, 75%, 80%, 85%, 90%, 93%, 94%, 95%, 96%, 97%, 98% or 99% and having antiviral activity.

For common rigid linkers used in the preparation of fusion proteins, in addition to the (EAAAK)n₁ sequence capable of forming an alpha-helix, another common rigid linker type is a proline (P)-rich sequence (XP)n₂, wherein X may be any amino acid, preferably alanine, lysine or glutamic acid. The (XP)n₂ sequence does not have a helical structure, but the proline can increase the rigidity of the backbone and effectively separate domains. Thus, in the present disclosure, the (EAAAK)n₁ linker can also be replaced by the (XP)n₂ linker without significantly affecting the antiviral activity. Therefore, the linker according to the present disclosure may be either (EAAAK)n₁ or (XP)n₂. Furthermore, in order to link to a lipophilic compound, the linker may also comprise one or more lysines (K) or cysteines (C).

The lipopeptides as described in the present disclosure may contain one or more chiral centers, and/or double bonds and structures like that, and thus may exist as stereoisomers, including isomers of double bonds (e.g., geometrical isomers), enantiomers (opticalisomeride), or diastereomers. Accordingly, any chemical structures within the scope as described here, whether partially or wholly containing the above similar structures, includes all possible enantiomers and diastereomers of the lipopeptides, including any one pure stereoisomer (e.g., pure geometrical isomers, pure enantiomers, or pure diastereomers), and any one mixture of these stereoisomers. One skilled in the art, by utilizing separation techniques or asymmetric synthesis methods, can also further resolve mixtures of these racemates and stereoisomers into the enantiomers or stereoisomers of the constituents thereof. The lipopeptides include, but are not limited to, various optical isomers, racemates and/or other mixtures. In the above case, a single enantiomer or diastereomer, e.g., an optically active isomer, can be obtained by an asymmetric synthesis method or a racemate resolution method. The resolution of racemates can be accomplished in a variety of ways, e.g., routine recrystallization with resolution-assisting reagents, or chromatography. In addition, the lipopeptides also include cis-and/or trans-isomers with double bonds.

The lipopeptides of the present disclosure include, but are not limited to, all of the various pharmaceutically acceptable forms of the lipopeptides. These pharmaceutically acceptable forms include various pharmaceutically acceptable salts, solvates, hydrates, complexes, chelates, non-covalent complexes, prodrugs based on the above substances, and any mixtures of the above forms.

The pharmaceutically acceptable salts of the present disclosure include acetate, lactobionate, benzenesulfonate, laurate, benzoate, malate, bicarbonate, maleate, bisulfate, mandelate, bitartrate, mesylate, borate, methylbromide, bromide, methylnitrate, calcium edetate, methylsulfate, camsylate, mucate, carbonate, napsylate, chloride, nitrate, clavulanate, N-methylglucamine, citrate, ammonium salt, dihydrochloride, oleate, edetate, oxalate, edisylate, pamoate, embonate, estolate, palmitate, esylate, pantothenate, fumarate, phosphate/diphosphate, gluceptate, polygalacturonate, gluconate, salicylate, glutamate, stearate, glycollylarsanilate, sulfate, hydroxybenzoate, subacetate, hydrabamine, succinate, hydrobromide, tannate, hydrochloride, tartrate, hydroxynaphthoate, teoclate, iodide, tosylate, triethiodide, lactate, and valerate, etc. Depending on the use, the pharmaceutically acceptable salt may be formed from cations such as sodium, potassium, and bismuth, or may be formed from a base such as ammonia, ethylenediamine, N-methyl-glutamine, lysine, arginine, ornithine, choline, N,N'-dibenzylethylenediamine, chloroprocaine, diethanolamine, procaine, diethylamine, piperazine, tris (hydroxymethyl) aminomethane and tetramethylammonium hydroxide. These salts may be prepared by standard methods, for example, by a reaction of a free acid with an organic or inorganic base. In the presence of a basic group (e.g., an amino group), an acid salt such as a hydrochloride, a hydrobromide, an acetate, and a pamoate may be used as a form of a drug; in the presence of an acidic group or an alcohol group, a pharmaceutically acceptable ester such as an acetate, a maleate, and a pivaloyloxymethyl, and an ester known in the literatures for improving solubility and hydrolyzability may be used as a form of sustained release drug or prodrug.

In another aspect, the present disclosure also provides an isolated nucleic acid, which encodes the above-described polypeptide or the variant thereof.

In another aspect, the present disclosure also provides a vector comprising the isolated nucleic acid. Vectors useful for inserting a polynucleotide of interest are well known in the art and include, but are not limited to, cloning vectors and expression vectors. In one embodiment, the vector is, for example, plasmid, cosmid, phage, etc.

In another aspect, the present disclosure also provides a host cell comprising the above-described isolated nucleic acid or the above-described vector. Such host cells include, but are not limited to, prokaryotic cells such as E. coli cells, and eukaryotic cells such as yeast cells, insect cells, plant cells, and animal cells (e.g., mammalian cells such as mouse cells and human cells). The host cell of the present disclosure may also be a cell line.

In another aspect, the present disclosure also provides a multimer formed from the above-described lipopeptide, a pharmaceutically acceptable salt, a solvate, a hydrate, a complex, a chelate, a non-covalent complex or a prodrug thereof, or the above-described polypeptide or the variant thereof.

In another aspect, the present disclosure also provides a conjugate comprising the above-described polypeptide or the variant thereof and a modification group. In some embodiments, the modification group is linked to the N-terminus or C-terminus of the polypeptide or the variant thereof, optionally through a linker. In some embodiments, the modification group is a terminal protecting group. The terminal protecting group of the polypeptide comprises an N-terminal protecting group and/or a C-terminal protecting group. As well known, the N-terminal protecting group may be any one selected from the group consisting of acetyl (Ac), amino (NH₂), maleoyl, succinyl, tert-butoxycarbonyl, benzyloxy, another hydrophobic group and macromolecular carrier group. As well known, the C-terminal protecting group may be any one selected from the group consisting of amino (NH₂), carboxyl, hydroxyl, amido, tert-butoxycarbonyl, another hydrophobic and macromolecular carrier group.

In another aspect, the present disclosure also provides a composition, comprising the above-described lipopeptide, a pharmaceutically acceptable salt, a solvate, a hydrate, a complex, a chelate, a non-covalent complex or a prodrug thereof, or the above-described polypeptide or the variant thereof, or the above-described conjugate, or the above-described isolated nucleic acid, or the above-described vector, or the above-described host cell, or the above-described multimer.

In another aspect, the present disclosure also provides a pharmaceutical composition, comprising the above-described lipopeptide, a pharmaceutically acceptable salt, a solvate, a hydrate, a complex, a chelate, a non-covalent complex or a prodrug thereof, and optionally further comprising a pharmaceutically acceptable carrier and/or excipient. In some embodiments, the lipopeptide, a pharmaceutically acceptable salt, a solvate, a hydrate, a complex, a chelate, a non-covalent complex or a prodrug thereof is present in an amount effective for treating a viral infection or a disease caused by viral infection.

In another aspect, the present disclosure also provides a pharmaceutical composition, comprising the above-described polypeptide or the variant thereof, optionally further comprising a pharmaceutically acceptable carrier and/or excipient. In some embodiments, the polypeptide or the variant thereof is present in an amount effective for treating a viral infection or a disease caused by viral infection.

In some embodiments, the viral infection is an infection caused by a virus selected from the group consisting of HIV and a drug-resistant strain thereof, SARS-CoV-2 and a mutant strain thereof, MERS-CoV, EBOV, MARV, IAV, RABV and VSV.

In some embodiments, the viral infection is an infection caused by HIV or a drug-resistant strain thereof.

In another aspect, the present disclosure also provides a method for treating a viral infection or a disease caused by viral infection, comprising administering an effective amount of the above-described lipopeptide or a pharmaceutical acceptable salt, a solvate, a hydrate, a complex, a chelate, a non-covalent complex or a prodrug thereof or the above-described polypeptide or the variant thereof or the above-described pharmaceutical composition to a subject in need thereof. In another aspect, the present disclosure also provides a method for inhibiting viral membrane fusion, comprising administering an effective amount of the above-described lipopeptide or a pharmaceutical acceptable salt, a solvate, a hydrate, a complex, a chelate, a non-covalent complex or a prodrug thereof or the above-described polypeptide or the variant thereof or the above-described pharmaceutical composition to a subject in need thereof. In some embodiments, the viral infection is an infection caused by a virus selected from the group consisting of HIV and a drug-resistant strain thereof, SARS-CoV-2 and a mutant strain thereof, MERS-CoV, EBOV, MARV, IAV, RABV and VSV. In some embodiments, the viral infection is an infection caused by HIV or a drug-resistant strain thereof.

In another aspect, the present disclosure also provides use of the above-described lipopeptide or a pharmaceutical acceptable salt, a solvate, a hydrate, a complex, a chelate, a non-covalent complex or a prodrug thereof or the above-described polypeptide or the variant thereof in the manufacture of a pharmaceutical composition, wherein the pharmaceutical composition is used as a viral membrane fusion inhibitor or for treating a viral infection or a disease caused by viral infection. In some embodiments, the viral infection is an infection caused by a virus selected from the group consisting of HIV and a drug-resistant strain thereof, SARS-CoV-2 and a mutant strain thereof, MERS-CoV, EBOV, MARV, IAV, RABV and VSV. In some embodiments, the viral infection is an infection caused by HIV or a drug-resistant strain thereof.

In another aspect, the present disclosure also provides the use of above-described lipopeptide or a pharmaceutical acceptable salt, a solvate, a hydrate, a complex, a chelate, a non-covalent complex or a prodrug thereof or the above-described polypeptide or the variant thereof, for use as a viral membrane fusion inhibitor or for use in treating a viral infection or a disease caused by viral infection. In another aspect, the present disclosure also provides the use of the above-described polypeptide or the variant thereof, for use as a viral membrane fusion inhibitor or for use in treating a viral infection or a disease caused by viral infection. In some embodiments, the viral infection is an infection caused by a virus selected from the group consisting of HIV and a drug-resistant strain thereof, SARS-CoV-2 and a mutant strain thereof, MERS-CoV, EBOV, MARV, IAV, RABV and VSV. In some embodiments, the viral infection is an infection caused by HIV or a drug-resistant strain thereof.

In another aspect, the present disclosure also provides a pharmaceutical composition for inhibiting viral membrane fusion or a pharmaceutical composition for treating a viral infection or a disease caused by viral infection, which comprises the above-described lipopeptide or a pharmaceutical acceptable salt, a solvate, a hydrate, a complex, a chelate, a non-covalent complex or a prodrug thereof or the above-described polypeptide or the variant thereof. In some embodiments, the viral infection is an infection caused by a virus selected from the group consisting of HIV and a drug-resistant strain thereof, SARS-CoV-2 and a mutant strain thereof, MERS-CoV, EBOV, MARV, IAV, RABV and VSV. In some embodiments, the viral infection is an infection caused by HIV or a drug-resistant strain thereof.

In some embodiments, the mutant strains of SARS-CoV-2 include Alpha mutant strains, Beta mutant strains, Gamma mutant strains, Epsilon mutant strains, Delta mutant strains, Omicron mutant strains, etc. In some embodiments, the mutant strains of SARS-CoV-2 include SARS-CoV-2 D614G and Omicron BA4/5 mutant strains. In some embodiments, the IAV includes Influenza A viruses, Influenza B viruses, and Influenza C viruses. In some embodiments, the Influenza A virus includes subtypes such as H1N1, H3N2, H5N1 and H7N9. In some embodiments, the Influenza A virus is H7N9.

In practice, the lipopeptide or polypeptide as described in the present disclosure may be administered to a patient as a medicament either directly or in admixture with a suitable carrier or excipient for the purpose of treating and/or preventing a viral (e.g., HIV, etc.) infection or a disease caused by viral infection. The material of the carrier includes, but is not limited to, water-soluble carrier material (e.g., polyethylene glycol, polyvinylpyrrolidone, organic acid, etc.), poorly soluble carrier material (e.g., ethyl cellulose, cholesterol stearate, etc.), enteric soluble carrier material (e.g., cellulose acetate phthalate, carboxymethyl cellulose, etc.), wherein the water-soluble carrier material is preferred. By using these materials, various preparation forms can be prepared, including but not limited to tablet, capsule, dripping pill, aerosol, pill, powder, solution, suspension, emulsion, granule, liposome, transdermal agent, buccal tablet, suppository, freeze-dried powder for injection, etc. The preparation form may be a conventional preparation, a sustained release preparation, a controlled release preparation and various microparticle delivery systems. In order to formulate a unit preparation form into a tablet, a wide variety of carriers known in the art may be used. Examples of carriers are, for example, diluent and absorbent, such as starch, dextrin, calcium sulfate, lactose, mannitol, sucrose, sodium chloride, glucose, urea, calcium carbonate, kaolin, microcrystalline cellulose, and aluminum silicate; wetting agent and binder, such as water, glycerol, polyethylene glycol, ethanol, propanol, starch slurry, dextrin, syrup, honey, glucose solution, gum arabic, gelatin paste, sodium carboxymethylcellulose, shellac, methylcellulose, potassium phosphate, and polyvinyl pyrrolidone; disintegrant, such as dried starch, alginate, agar powder, brown algae starch, sodium bicarbonate and citric acid, calcium carbonate, polyoxyethylene, sorbitol fatty acid ester, sodium dodecylsulfate, methyl cellulose, and ethyl cellulose; disintegration inhibitor, such as sucrose, glyceryl tristearate, cocoa butter, and hydrogenated oil; absorption promoters, such as quaternary ammonium salts, and sodium lauryl sulfate; lubricant, such as talc, silica, corn starch, stearate, boric acid, liquid paraffin, and polyethylene glycol. The tablet may also be further formulated into a coated tablet, such as sugar-coated tablet, film-coated tablet, enteric-coated tablet, or double or multiple layer tablet. In order to formulate a unit preparation form into a pill, a wide variety of carriers known in the art may be used. Examples of the carrier are, for example, diluent and absorbent, such as glucose, lactose, starch, cocoa butter, hydrogenated vegetable oil, polyvinyl pyrrolidone, Gelucire, kaolin, and talc; binder, such as gum arabic, tragacanth, gelatin, ethanol, honey, liquid sugar, rice paste, and flour paste; disintegrant, such as agar powder, dried starch, alginate, sodium dodecyl sulfate, methyl cellulose, and ethyl cellulose. In order to formulate a unit preparation form into a suppository, a wide variety of carriers known in the art may be used. Examples of the carrier are, for example, polyethylene glycol, lecithin, cocoa butter, higher alcohol, higher alcohol ester, gelatin, and semisynthetic glyceride. In order to formulate a unit preparation form into a preparation for injection such as a solution, an emulsion, a freeze-dried powder and a suspension, all conventional diluents may be used, for example, water, ethanol, polyethylene glycol, 1,3-propanediol, ethoxylated isostearyl alcohol, polyoxylated isostearyl alcohol, and polyoxyethylene sorbitan fatty acid ester. In addition, in order to prepare an isotonic injection, to a preparation for injection, sodium chloride, glucose, or glycerin in a suitable amount may be added, and furthermore, conventional cosolvent, buffer, pH-adjusting agent may also be added. Besides, if required, coloring agent, preservative, perfume, flavor, sweeting agent, and other material may optionally be added to the pharmaceutical preparation. The above preparation forms may be administered by injection including subcutaneous injection, intravenous injection, intramuscular injection, intracavitary injection and the like, intraluminal administration such as transrectal and vaginal administration, respiratory administration such as nasal administration; and mucosal administration. Among the above administration routes, the administration by injection is preferred.

The administration dose of the lipopeptide or polypeptide of the present disclosure depends on various factors, for example, the nature and severity of a disease to be prevented or treated, the gender, age, weight and individual response of a patient or animal, a specific ingredient used, an administration route, and an administration frequency etc. The above dose may be administered in a single-unit dosage form or multiple-(e.g., two, three or four) unit dosage forms.

The lipopeptide or polypeptide of the present disclosure may be directly used alone for the treatment or prevention of a viral (e.g., HIV, etc.) infection or a disease caused by viral infection, or may be used in combination with one or more antiviral drugs, to achieve the purpose of improving overall therapeutic effects. The antiviral drugs include, but are not limited to, reverse transcriptase inhibitors, protease inhibitors, entry inhibitors, integration inhibitors, maturation inhibitors, and the like. The above reverse transcriptase inhibitors may be one or more inhibitors selected from the group consisting of AZT, 3TC, ddI, d4T, ddT, TDF, Abacavir, Nevirapine, Efavirenz, Delavirdine, Azvudine, Ainuovirine and the like; the above protease inhibitors may be one or more inhibitors selected from the group consisting of Saquinavir mesylate, Indinavir, Ritonavir, Amprenavir, Kaletra, Nelfinavir mesylate and the like; the above invasion inhibitors may be one or more inhibitors selected from the group consisting of Maraviroc, TAK-779, T20, T2635, Sifuvirtide, Albuvirtide, and the like; the above integration inhibitors may be one or more inhibitors selected from the group consisting of Raltegravir, Dolutegravir, Elvitegravi and the like.

A specific therapeutically effective dose level for any individual patient will depend on various factors, including disorder being treated and the severity thereof; the activity of specific active ingredient used; a specific composition used; the age, weight, general health, gender and diet of a patient; an administration time, an administration route and an excretion rate of a specific active ingredient used; a duration of treatment; a drug used together with the specific active ingredient used in combination or simultaneously; and similar factors well known in the medical field. For example, it is common practice in the art to start with a dosage of an active ingredient at a level below that required to achieve a desired therapeutic effect, and to gradually increase the dosage until the desired effect is achieved. In general, the lipopeptide or polypeptide of the present disclosure may be administered to a mammal, particularly a human, in a dosage of between 0.001 mg/kg body weight/day and 1000 mg/kg body weight/day, such as between 0.01 mg/kg body weight/day and 100 mg/kg body weight/day, further such as between 0.1 mg/kg body weight/day and 10 mg/kg body weight/day.

### Brief Description of the Drawings

FIG. 1 is a diagram showing the sequence structures of T20, C34, T1249, T2635, SFT, ABT, C34-Chol, LP-11 and LP-98, where A is a diagram showing the structure and function of HIV-1 fusion protein gp41; B is a diagram of polypeptides, wherein underlined sequences are PBD, three amino acids inserted into the NHR pocket are labeled with red color, and the mutant amino acids are labeled with green color; Chol represents cholesterol molecules and C₁₆ represents palmitic acid molecules.
FIG. 2 is a diagram showing the sequence structures of the viral membrane fusion inhibitors (including LP-121, LP-122, LP-123, LP-124, LP-125, LP-126, LP-127 and LP-128) provided in the examples of the present disclosure, wherein underlined sequences are PBD, three amino acids inserted into the NHR pocket are labeled with red color, and the mutant amino acids are labeled with green color; Chol in the brackets represents cholesterol molecules.

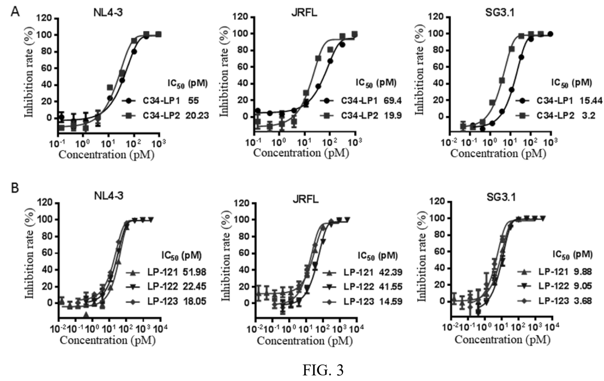

FIG. 3 shows anti-HIV activity of the viral membrane fusion inhibitors provided in the examples of the present disclosure, wherein A shows the anti-HIV activity comparisons between C34-LP1 and C34-LP2; B shows the anti-HIV activity comparisons of LP-121, LP-122 and LP-123, the used HIV-1 strains NL4-3 and JRFL are pseudoviruses, and the SG3.1 is a replicative virus; the used target cell is a TZM-bl cell.

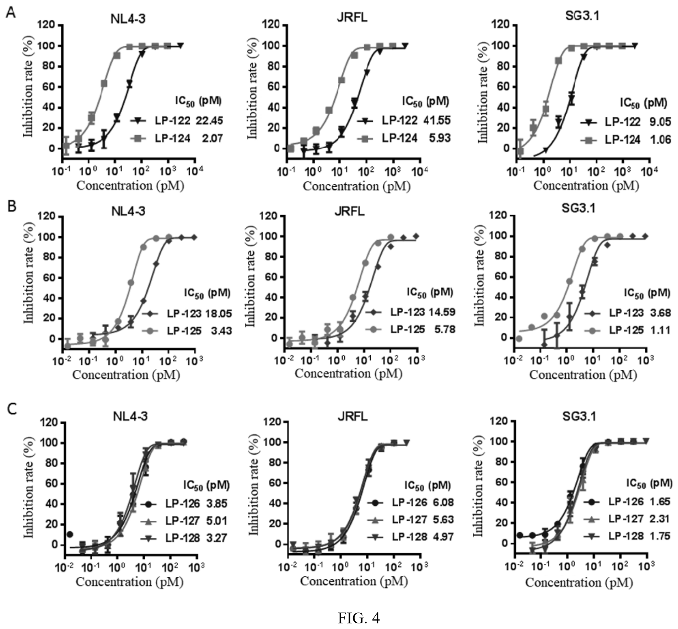

FIG. 4 shows anti-HIV activity of the viral membrane fusion inhibitors provided in the examples of the present disclosure, where A shows the anti-HIV activity comparisons between LP-122 and LP-124; B shows the anti-HIV activity comparisons between LP-123 and LP-125, C shows the anti-HIV activity comparisons between LP-126, LP-127 and LP-128, the used HIV-1 strains NL4-3 and JRFL are pseudoviruses, and the SG3.1 is a replicative virus; the used target cell is a TZM-bl cell.

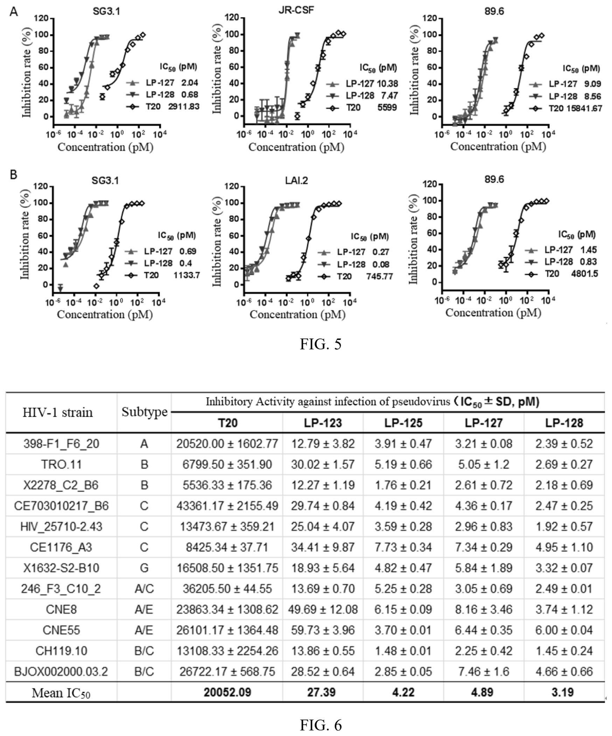

FIG. 5 shows the anti-HIV activity comparisons of LP-127 and LP-128 provided in the examples of the present disclosure with T20, wherein A shows their inhibitory activities against replicative HIV-1 stains SG3.1, JR-CSF and 89.6 to infect TZB-bI cells; B shows their inhibitory activity against replicative HIV-1 stains SG3.1, LAI.2 and 89.6 to infect MT-4 cells.
FIG. 6 shows the inhibitory activities of the T20 and the LP-123, LP-125, LP-127, and LP-128 provided in the examples of the present disclosure against representative pandemic HIV-1 strain pseudoviruses.

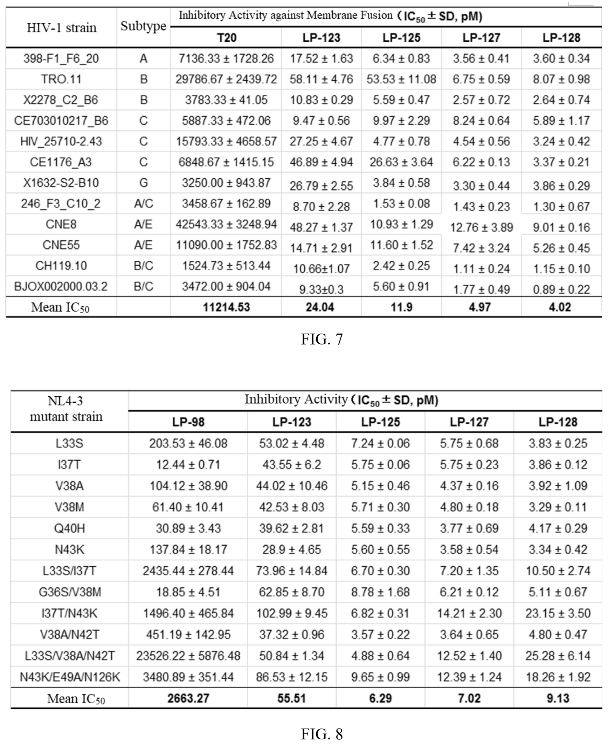

FIG. 7 shows the inhibitory activities of the T20 and the LP-123, LP-125, LP-127, and LP-128 provided in the examples of the present disclosure against cell fusion mediated by representative pandemic HIV-1 strains.
FIG. 8 shows the inhibitory activities of the LP-98 and the LP-123, LP-125, LP-127, and LP-128 provided in the examples of the present disclosure against drug-resistant HIV-1 stains.

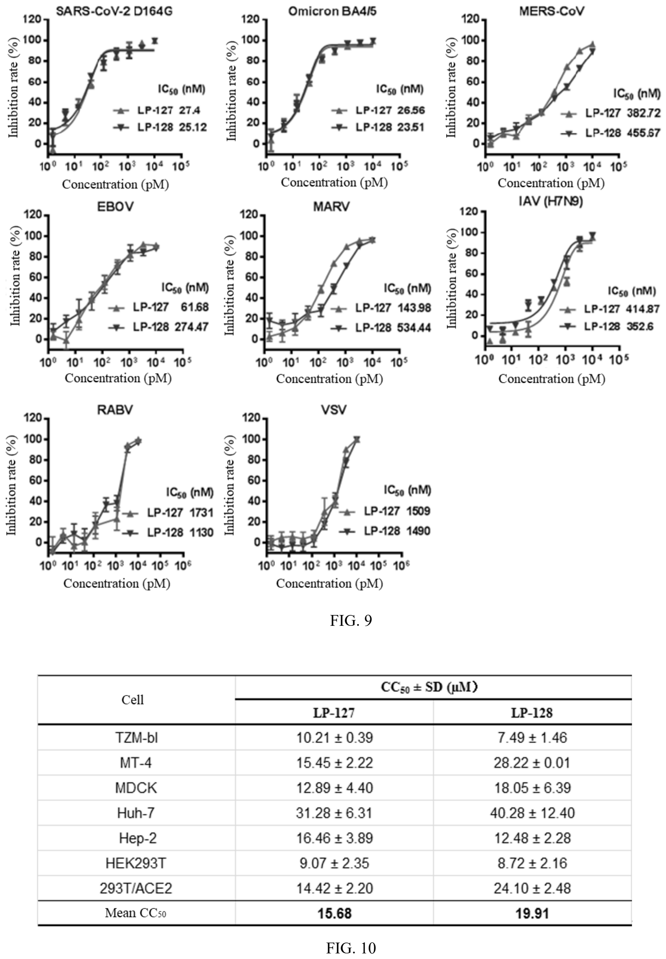

FIG. 9 shows the broad-spectrum antiviral activity evaluation results of LP-127 and LP-128 provided in the examples of the present disclosure, wherein the inhibitory activities against SARS-CoV-2, MERS-CoV, EBOV, MARV, IAV (H7N9), RABV, and VSV each are evaluated with pseudovirus systems.
FIG. 10 shows the in vitro cytotoxicity evaluation of LP-127 and LP-128 provided in the examples of the present disclosure.

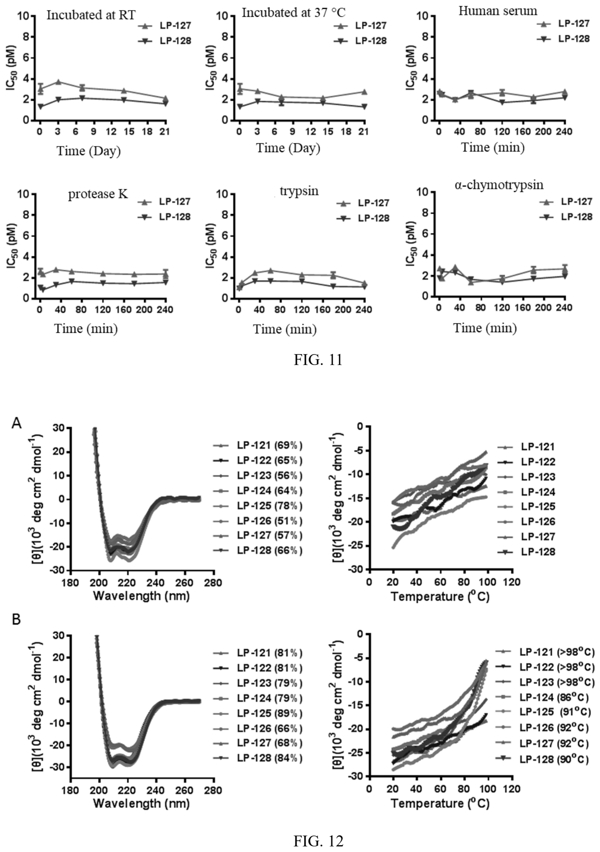

FIG. 11 shows the stability analysis results of LP-127 and LP-128 provided in the examples of the present disclosure.
Fig. 12 shows the secondary structure and thermal stability of the membrane fusion inhibitors provided in the examples of the present disclosure as detected by using circular dichroism (CD), wherein A shows the alpha-helix content (left figure) and thermal stability (left figure) of the lipopeptides alone; B shows alpha-helix content (left figure) and thermal stability (left figure) of the complex formed between the lipopeptide and the target sequence polypeptide N44.

### Sequence Information

Information on the sequences involved in the present disclosure is provided in the following Table 1.

**Table 1: Descriptions of sequences**

| SEQ ID NO. | Names/ description | Sequence information |
|---|---|---|
| 1 | T20 | Ac-YTSLIHSLIEESQNQQEKNEQELLELDKWASLWNWF-NH₂ |
| 2 | C34 | Ac-**W**ME**W**DRE**I**NNYTSLIHSLIEESQNQQEKNEQELL-NH₂ |
| 3 | T1249 | Ac-**W***Q*E**W***EOK***I***TA*L*L*E*QAQI*QQEKNE*Y*EL*QK*LDKWASLWEWF-NH₂ |
| 4 | T2635 | Ac-TT**W***EA***W**DRA**I***AE*Y*AA*R*IE*ALI*RAA*Q*E*QQEKNE*AA*L*R*EL-NH₂ |
| 5 | SFT | Ac-S**W***ET***W***E*RE**I***E*NYT*RO*I*YR*I*L*EESQ*E*QQ*DR*NE*RD*LLE-NH₂ |
| 6 | ABT | Ac-**W***E*E**W**DRE**I**NNYT*K*LIH*E*LIEESQNQQEKNEQELL-NH₂ |
| 7 | C34-Chol | Ac-WMEWDREINNYTSLIHSLIEESQNQQEKNEQELL-GSG-C(Chol)- NH2 |
| 8 | LP-11 | Ac-*E*MT**W***E*E**W***EKK***I***EE*YT*KK*I*EEILK*-PEG₈-K(C₁₆)-NH₂ |
| 9 | LP-98 | Ac-YE*QK*I*EE*L*LKKAEE*QQ*K*KNE*E*L*KK*L*E*K(Chol)-NH₂ |
| 10 | C34-LP1 | Ac- WMEWDREINNYTSLIHSLIEESQNQQEKNEQELLGSGSGK(Chol)- NH2 |
| 11 | C34-LP2 | Ac- WMEWDREINNYTSLIHSLIEESQNQQEKNEQELLEAAAKK(Chol)- NH2 |
| 12 | LP-121 | Ac- EMTWEEWEKKIEELEKKIEELLKKAEEQQKKNEAAAKK(Chol)- NH2 |
| 13 | LP-122 | Ac-TWEEWEKKIEELEKKIEELLKKAEEQQKKNEQEAAAKK(Chol)- NH2 |
| 14 | LP-123 | Ac-T**W***E*E**W***EKK***I***EELEKK*I*EE*L*LKKAEE*QQ*K*KNEAAAKK(Chol)-NH₂ |
| 15 | LP-124 | Ac-T***Y**E*E***Y**EKK***I***EELEKK*I*EE*L*LKKAEE*QQ*K*KNEQEAAAKK(Chol)-NH₂ |
| 16 | LP-125 | Ac*-T****Y**E*E***Y**EKK*I*EELEKK*I*EE*L*LKKAEE*QQ*K*KNEAAAKK(Chol)-NH₂ |
| 17 | LP-126 | Ac*-E****Y**E*E***Y**EKK***I***EELEKK*I*EE*L*LKKAEE*QQ*K*KNEAAAKK(Chol)-NH₂ |
| 18 | LP-127 | Ac-*E****Y**E*E***Y**EK*E***L**EELEKK*L*LKKAEE*QQ*K*KNEAAAKK(Chol)-NH₂ |
| 19 | LP-128 | Ac-E***Y**EK*E***L**EKKIEEL*L*KKAEE*QQ*K*KNEAAAKK(Chol)-NH₂ |
| 20 | N44 | Ac-TVQARQLLSGIVQQQNNLLRAIEAQQHLLQLTVWGIKQLQARIL- NH2 |
| 21 | Polypeptide 1 | Ac-**W**ME**W**DRE**I**NNYTSLIHSLIEESQNQQEKNEQELLEAAAKK-NH₂ |
| 22 | Polypeptide 2 | Ac-EMT**W***E*E**W***EKK***I***EELEKK*I*EE*L*LKKAEE*QQ*K*KNEAAAKK-NH₂ |
| 23 | Polypeptide 3 | Ac-T**W***E*E**W***EKK*I*EELEKK*I*EE*L*LKKAEE*QQ*K*KNEQEAAAKK-NH₂ |
| 24 | Polypeptide 4 | Ac-T**W***E*E**W***EKK***I***EELEKK*I*EE*L*LKKAEE*QQ*K*KNEAAAKK-NH₂ |
| 25 | Polypeptide 5 | Ac-T***Y**E*E***Y**EKK***I***EELEKK*I*EE*L*LKKAEE*QQ*K*KNEQEAAAKK-NH₂ |
| 26 | Polypeptide 6 | *Ac-*T***Y**E*E***Y**EKK***I***EELEKK*I*EE*L*LKKAEE*QQ*K*KNEAAAKK-NH₂ |
| 27 | Polypeptide 7 | Ac*-E****Y**E*E***Y**EKK***I***EELEKK*I*EE*L*LKKAEE*QQ*K*KNEAAAKK-NH₂ |
| 28 | Polypeptide 8 | Ac-*E****Y**E*E***Y**EK*E***L**EELEKK*I*EE*L*LKKAEE*QQ*K*KNEAAAKK-NH₂ |
| 29 | Polypeptide 9 | Ac-E***Y**EK*E***L**EELEKKIEE*L*LKKAEE*QQ*K*KNEAAAKK-NH₂ |

| | | |
|---|---|---|
| Note: underlined sequences are PBD, three amino acids inserted into the NHR pocket are labeled in bold, and mutated amino acids are labeled in italics; Chol represent cholesterol molecule, and C₁₆ represents palmitic acid molecules. | | |

### Examples

The present disclosure will be described in detail below with reference to the specific embodiments, and the examples provided here are only for illustrating the present disclosure but not for limiting the scope of the present disclosure. The examples provided below serve as a guidance for one of ordinary skill in the art to make modifications, but not construct any limits on the present disclosure in any way. One skilled in the art can refer to the present disclosures to appropriately modify related parameters. Specifically, it is necessary to indicate that all similar substitutions or changes are obvious for one skilled in the art, and all of them are considered to be within the scope of the present disclosure. The method of the present disclosure has been described with preferred examples, and it will be apparent that related person can achieve and apply the techniques of the present disclosure by making modifications or appropriate changes or combinations to the compounds and preparation methods as described herein, without departing from the contents, spirits and scope of the present disclosure.

The experimental methods in the following examples, unless otherwise illustrated, each are conventional methods, and they are carried out according to the techniques or conditions described in literature in this field or according to the product instructions. The materials, reagents and the like used in the following examples, unless otherwise illustrated, each are commercially available.

### Example 1: Preparation of Viral Membrane Fusion Inhibitor Lipopeptides

The cholesterol modifications of ten lipopeptides (C34-LP1, C34-LP2, LP-121, LP-122, LP-123, LP-124, LP-125, LP-126, LP-127 and LP-128) prepared in the examples of the present disclosure each were achieved by the amidation reaction between a cholesteryl bromoacetate and the side chain amino group of the C-terminal lysine (K) of the peptide chain, and the specific method was described in the literature published by the inventors' laboratory^{[7]}. The amino terminals of all the lipopeptides prepared in the examples of the present disclosure each linked an acetyl (Ac) as an amino-terminal protecting group, and the carboxyl terminals each linked an amino (NH₂) as a carboxy-terminal protecting group.

### I. Chemical Reagents Required in Preparation Process

All chemical reagents, such as various Fmoc amino acids, N,N'-diisopropylcarbodiimide (DIC), 1-hydroxybenzotriazole (HOBt), N,N-dimethylformamide (DMF), piperidine (PIPE), ninhydrin, acetic anhydride (Ac₂O), N,N-diisopropylethylamine (DIEA), hydrazine hydrate, cholesteryl hemisuccinate, trifluoroacetic acid (TFA), ethanedithiol (EDT), thioanisole (TA), triisopropylsilane (TIPS), phenol and the like, each were purchased from major chemical reagent suppliers and were not further purified prior to use. Amino acid-protecting raw materials used in the synthesis of polypeptides included Fmoc-Lys(Dde)-OH, Fmoc-Lys(Boc)-OH, Fmoc-Trp(Boc)-OH, Fmoc-Met-OH, Fmoc-Thr(tBu)-OH, Fmoc-Glu(OtBu)-OH, Fmoc-Asp(OtBu)-OH, Fmoc-Arg(Pbf)-OH, Fmoc-Ile-OH, Fmoc-Asn(Trt)-OH, Fmoc-Tyr(tBu)-OH, Fmoc-Ser(tBu)-OH, Fmoc-Leu-OH, Fmoc-Gln(Trt)-OH, Fmoc-Gly-OH, and Fmoc-Ala-OH. The abbreviations among them have well known meanings, for example: Fmoc is 9-fluorenylmethoxycarbonyl, Dde is 1-(4,4-dimethyl-2,6-dioxocyclohexylene) ethyl, Boc is t-butoxycarbonylacyl, tBu is t-butyl, OtBu is t-butoxy, Trt is trityl, and Pbf is (2,3-dihydro-2,2,4,6,7-pentamethylbenzofuran-5-yl)sulfonyl.

### II. Synthesis of Peptide Resins

Rink Amide MBHA resin was used as a carrier resin and it was sequentially coupled with corresponding protected amino acids in the amino acid sequences of polypeptides through Fmoc deprotection and coupling reaction, to prepare peptide resins.

### 1. Coupling of the First Protected Amino Acid to Main Chain

0.3 mmol of the first protected amino acid Fmoc-Lys(Dde)-OH and 0.3 mmol of HOBt were taken and dissolved with an appropriate amount of DMF; 0.3 mmol of DIC was additionally taken, slowly added into the DMF solution of the protected amino acid under shaking, and the resulting mixture was reacted at room temperature for 5 minutes under shaking, to obtain an activated protected amino acid solution, which was set aside for later use.

0.1 mmol of Rink Amide MBHA resin (0.35 mmol/g×0.3 g) were taken and deprotected with a 25% PIPE/DMF solution (volume ratio) for 20 min (twice), after washing and filtering, to obtain an Fmoc-removed resin.

The activated first protected amino acid solution was added into the Fmoc-removed resin to carry out a coupling reaction for 60 minutes, after filtering and washing, to obtain a resin containing the first protected amino acid Fmoc-Lys(Dde).

### 2. Coupling of Other Protected Amino Acids to Main Chain

By using the same method as above for coupling the first protected amino acid to the main chain, the corresponding other protected amino acids of the polypeptide were sequentially coupled, to obtain resins containing main chain amino acids. At last, the N-terminus was capped by acetylation with 0.3 mmol of Ac₂O and 0.6 mmol of DIEA, to complete the synthesis of the main chain. After the reaction in each of the above steps, the reaction was controlled by Kaiser Test, and if the condensation reaction of certain amino acid was incomplete, the condensation was repeated once until the desired target peptide segment was obtained, i.e., the polypeptide as described in the present disclosure.

### 3. Cholesterol Coupling of Lysine Side Chains

The resin was treated with 2% hydrazine hydrate/DMF solution (volume ratio) in a volume as small as possible to remove the side chain Dde protecting group of the C-terminal lysine (for 10 minutes, twice), after filtering and washing, to obtain a Dde-removed resin, which was set aside for later use. 0.3 mmol of cholesteryl hemisuccinate and 0.3 mmol of HOBt were taken and dissolved with an appropriate amount of DMF; 0.3 mmol of DIC was additionally taken, slowly added into the DMF solution of the cholesteryl hemisuccinate and HOBt, and the resulting mixture was reacted at room temperature for 5 minutes under shaking. The prepared solution comprising cholesteryl hemisuccinate, HOBt and DIC was added to the obtained Dde-removed resin to carry out a coupling reaction for 60 minutes, after filtering, washing and drying, to obtain a peptide resin.

### III. Preparation of Crude Products

The above peptide resin was taken and added into a cleavage reagent (the cleavage agent 15 mL/g resin), and mixed well, the resulting mixture was reacted at 30 °C for 3 hours under shaking, thereby cleaving the polypeptide of interest from the resin and removing the side chain protecting group. The filtrate of the reaction mixture was collected. The resin was further washed with a small amount of TFA/DCM for three times, and after the filtrates were combined, the combined filtrates was precipitated by adding anhydrous ether and centrifuged. The filter cake was further washed and precipitated with cold anhydrous ether for second times, then dried under vacuum, to obtain white powder, i.e., the lipopeptide crude product; the cleavage reagent had the following composition: trifluoroacetic acid : 1,2-ethanedithiol : thioanisole : phenol : H₂O : triisopropylsilane = 68.5 : 10 : 10 : 5 : 3.5 : 1 (v/v).

### IV. Preparation of Pure Products

The above lipopeptide crude product was taken and dissolved by adding water/acetonitrile, and the resulting mixture was centrifuged to remove insoluble substances, and was set aside for later use. A reversed-phase high performance liquid chromatograph was used for purification. The used chromatographic column was Agela C18 (10 µm, 100Å, 50×250 mm), with a mobile phase consisting of a mobile phase A (0.05% TFA and 2% acetonitrile in water) and a mobile phase B (90% acetonitrile in water). The mobile phase had a flow rate of 25 mL/min, and the ultraviolet detection wavelength was 220 nm. The crude product solution was loaded on the chromatographic column to perform a gradient elution, and the corresponding purified components were collected and directly lyophilized to remove solvents, to obtain a pure product of trifluoroacetate of polypeptide in a fluffy state.

The pure product of trifluoroacetate of polypeptide was re-dissolved with water and acetonitrile, to which a large amount of anion exchange resin (in the form of an acetate radical) was added, the resulting mixture was stirred for 3 hours, and filtered, and then the ion exchange resin was rinsed with a water/acetonitrile mixed solvent, the filtrates were combined and lyophilized, to obtain a pure product of polypeptide acetic acid salt in a fluffy state.

The chemical structure of the synthesized lipopeptide was characterized by MALDI-TOF mass spectrometry, and the purity was determined by an analytical high performance liquid chromatography (Agela C18-4.6×250 mm, flow rate: 1 mL/min). The results showed that the synthesized lipopeptides had a purity of more than 95%.

### Example 2 Effect of EAAAK Rigid Linkers on Anti-HIV Activity of Membrane Fusion Inhibitors

The present example compared C34-LP1 (containing a GSGSG flexible linker) and C34-LP2 (containing an EAAAK rigid linker) in the anti-HIV activity, and the used materials and methods were described in the literature published by the inventors' laboratory^{[7]}, where the used viruses were pseudoviruses of HIV-1 strains NL4-3 and JRFL and replicative viruses (living viruses) of HIV-1 strain SG 3.1.

### 1. Experimental Method

(1) Preparation of Viruses: plasmids expressing NL4-3 or JRFL envelope protein (Env) were co-transfected with HIV-1 backbone plasmids pSG3Δenv into HEK293T cells; molecular clones encoding SG3.1 were transfected into HEK293T cells. The transfected cells were cultured in a cell culture incubator at 37 °C and 5% CO₂ for 48 hours, and then the supernatant was collected and filtered to collect the filtrate, to be a virus solution containing NL4-3 pseudoviruses or JRFL pseudoviruses or SG3.1 living viruses, after titration, which was preserved at -80 °C for later use.
(2) The test lipopeptides were dissolved in deionized water and then diluted with a DMEM medium in a 3-fold serial dilution to obtain a lipopeptide diluent. 9 dilutions were set for each test lipopeptide.
(3) In a 96-well plate, the lipopeptide diluent (50 µL/well) was added to drug wells and the DMEM medium (50 µL/well) was added to control wells. Each well was set with three duplicate wells. Then, a 100 TCID₅₀ virus solution (which was adjusted to be 50 µL/well) was added and incubated at room temperature for 30 minutes.
(4) TZM-b1 cells were re-suspended in a DMEM medium and DEAE-dextran was added thereto such that the cell concentration was about 10×10⁴ cells/mL and the DEAE-dextran concentration was 15 µg/mL. After the step (3) was finished, a TZM-b1 cell suspension (100 µL/well) was added to the 96-well plate and cultured for 48 hours in a cell culture incubator at 37 °C and 5% CO₂.
(5) The cell culture supernatant was discarded and 30 µL of a cell lysate (Promega, Cat. No.: E1531) was added to each well. The cell lysis was carried out for 15 minutes at room temperature, and then a luciferase assay substrate reagent (Promega, Cat. No.: E1501) was added. Relative luminescence units (RLU) were measured by a microplate luminometer, an inhibition rate curve was made, and the half-maximal inhibition concentration (IC₅₀) of the drug was calculated.

### 2. Experimental Results and Analysis

The results were shown in FIG. 3A. The results showed that the C34-LP1 inhibited NL4-3 pseudovirus and JRFL pseudovirus to infect the TZM-bl cells with average IC₅₀ values of 55 pM and 69.4 pM, respectively, while the C34-LP2 inhibited NL4-3 pseudovirus and JRFL pseudovirus to infect the TZM-bl cells with average IC₅₀ values of 20.23 pM and 19.9 pM respectively; the C34-LP1 inhibited SG3.1 living virus to infect the MT-4 cells with average IC₅₀ value of 15.44 pM, while the C34-LP2 inhibited SG3.1 living virus to infect the MT-4 cells with average IC₅₀ value of 3.2 pM. By comparison, the inhibitory activity of the C34-LP2 against NL4-3 pseudovirus and JRFL pseudovirus was higher than that of the C34-LP1 by about 3 folds and 4 folds, respectively; the inhibitory activity of the C34-LP2 against SG3.1 living virus was higher than that of the C34-LP1 by about 5 folds. The experimental results showed that the EAAAK rigid linkers would help to improve the anti-HIV activity of membrane fusion inhibitors.

### Example 3 Design and Antiviral Activity Identification of Membrane Fusion Inhibitors

By directly comparing the anti-HIV activity of C34-LP1 and C34-LP2 in, it was confirmed that the use of an EAAAK rigid linker can improve the antiviral effect of lipopeptide membrane fusion inhibitors. The examples of the present disclosure further prepared the lipopeptides LP-121, LP-122 and LP-123, in which EAAAK was used as a linker, and EE and KK amino acid pairs further were introduced at the corresponding positions *b, c* and *f, g* of the CHR helix respectively, to promote the formation of a "salt bridge" structure. By utilizing the method in Example 2, the anti-HIV activity of LP-121, LP-122 and LP-123 were determined. The results were shown in FIG. 3B. The results showed that the IC₅₀ values of LP-121 against NL4-3 pseudovirus, JRFL pseudovirus and SG3.1 living virus were 51.98 pM, 42.39 pM and 9.88 pM, respectively; the IC₅₀ values of LP-122 against NL4-3 pseudovirus, JRFL pseudovirus and SG3.1 living virus were 22.45 pM, 41.55 pM and 9.05 pM, respectively; the IC₅₀ values of LP-123 against NL4-3 pseudovirus, JRFL pseudovirus and SG3.1 living virus were 18.05 pM, 14.59 pM and 3.68 pM, respectively. By comparison, it can be found that the LP-123 lipopeptide containing a shorter sequence exhibited optimal anti-HIV activity, while the LP-121 lipopeptide with a prolonged N-terminus and the LP-122 lipopeptide with a prolonged C-terminus showed decreased anti-HIV activity. This unexpected result revealed the relationship between the structure and function of the membrane fusion inhibitors.

### Example 4 Technical Strategy of Further Optimizing Membrane Fusion Inhibitors

In order to further improve the antiviral activity of the membrane fusion inhibitors, the present disclosure creatively prepared the membrane fusion inhibitor lipopeptides LP-124 and LP-125. The two hydrophobic amino acids W of the N-terminal pocket-binding domain (PBD) in the polypeptide sequences of the LP-122 and the LP-123 were substituted with two hydrophilic and small molecular weight amino acids Y, to promote the conversion of the interaction between the inhibitors and the NHR target in the pocket domain from highly hydrophobicity to hydrophilicity.

Likewise, by utilizing the experimental method in Example 2, the anti-HIV activities of the LP-124 and LP-125 were determined and compared for analysis.

The results were shown in FIG. 4A and FIG. 4B. The results showed that the the IC₅₀ values of LP-124 against NL4-3 pseudovirus, JRFL pseudovirus and SG3.1 living virus were 2.07 pM, 5.93 pM and 1.06 pM, respectively, which were increased as compared to the activity of its template LP-122 by about 11 folds, 7 folds and 9 folds, respectively. The IC₅₀ values of LP-125 against NL4-3 pseudovirus, JRFL pseudovirus and SG3.1 living virus were 3.43 pM, 5.78 pM and 1.11 pM, respectively, which were increased as compared to the activity of its template LP-123 by about 5 folds, 3 folds and 3 folds, respectively.

Further, the examples of the present disclosure prepared three lipopeptides LP-126, LP-127 and LP-128, and their anti-HIV activities were determined by using the same method as above described. The results were shown in FIG. 4C. The results showed that the IC₅₀ values of LP-126, LP-127 and LP-128 against NL4-3 pseudovirus were 3.85 pM, 5.01 pM and 3.27 pM, respectively; the the IC₅₀ values of LP-126, LP-127 and LP-128 against JRFL pseudovirus were 6.08 pM, 5.63 pM and 4.97 pM, respectively; the IC₅₀ values of LP-126, LP-127 and LP-128 against SG3.1 living virus were 1.65 pM, 2.31 pM and 1.75 pM, respectively.

These results showed that in the PBD sequence of membrane fusion inhibitors, the substitution of W amino acid with Y amino acid could significantly increase the antiviral activity.

### Example 5 Comparison and Analysis of the Anti-HIV Activity of LP-127, LP-128 and T20

T20 is currently the only one membrane fusion inhibitor that has been approved for clinical use by U.S Food and Drug Administration (FDA) for treatment of HIV-1 infection. In the present example, multiple replicative HIV-1 strains were used to further evaluate the anti-HIV activities of LP-127 and LP-128, and their inhibitory activities were compared with that of the T20.

### 1. Experimental Method

The preparation method for the replicative HIV-1 strains SG3.1, JRCSF, 89.6 and LAI.2 was the same as those described in Example 2, which were obtained by transfecting molecular clone plasmids into HEK293T cells, and the specific method was described in the literature published by the inventors' laboratory^{[7]}. The examples of the present disclosure not only determined the activity of the membrane fusion inhibitors inhibiting these viruses to infect TZB-bI cells(the method was the same as that in Example 2), but also determined the activity of the membrane fusion inhibitors inhibiting these replicative HIV-1 strains to infect MT-4 cells, with the specific experimental method below:
(1) The lipopeptides were gradually diluted in a 96-well plate, by setting 3 replicate wells and 9 gradients, and then 200 TCID₅₀ viruses and about 4×10⁴ MT-4 cells were added to each well in order.
(2) The plate was placed in an incubator at 37 °C and 5% CO₂ for 4 hours, then was centrifuged at 200 g at room temperature for 5 minutes and then the supernatant was discarded. The cells were further washed once with RPMI-1640 culture medium at 200 µl/well, and then RPMI-1640 complete culture medium at 200 µl/well was add to culture the cells.
(3) On the fourth day, the 96-well plate was centrifuged at room temperature at 200 g for 5 min, and the supernatant was taken at 30 µl/well and transferred into a new 96-well plate, to which 1% Triton-X100 solution was added at 120 µl/well for inactivating the viruses. Positive control wells and the initial concentration were used for P24 antigen quantification to determine the dilution factor.. The P24 antigen quantification was performed according to the operating method as described in the specification of the kit, using a 96-well microplate reader for measurement. Then, each culture supernatant was diluted to an appropriate concentration to conduct the P24 antigen quantitative assay. The inhibition curve for each drug was fitted by using the four-parameter logistic equation in non-linear regression analysis and the IC₅₀ values were calculated.

### 2. Experimental Results and Analysis

The results were shown in FIG. 5.

In the TZM-bl cells, LP-127, LP-128 and T20 inhibited SG3.1 with the IC₅₀ values of 2.04 pM, 0.68 pM and 2911.83 pM, respectively, inhibited JR-CSF with the IC₅₀ values of 10.38 pM, 7.47 pM and 5599 pM, respectively, and inhibited 89.6 with the IC₅₀ values of 9.09 pM, 8.56 pM and 15841.67 pM, respectively (in FIG. 5A). By comparison, the inhibitory activities of the LP-127 and LP-128 against SG3.1 were higher than that of the T20 by 1427 folds and 4282 folds, respectively; the activities for inhibiting JR-CSF were higher than the T20 by 539 folds and 750 folds, and their inhibitory activities against 89.6 were higher than that of the T20 by 1743 folds and 1851 folds inhibitory, respectively.

In the MT-4 cells, LP-127, LP-128 and T20 inhibited SG3.1 with the IC₅₀ values of 0.69 pM, 0.4 pM and 1133.7 pM, respectively, inhibited LAI.2 with the IC₅₀ values of 0.27 pM, 0.08 pM and 745.77 pM, respectively, and inhibited 89.6 with the IC₅₀ values of 1.45 pM, 0.83 pM and 4801.5 pM respectively (in FIG. 5B). By comparison, the inhibitory activities of the LP-127 and LP-128 against SG3.1 were higher than that of the T20 by 1643 folds and 2834 folds, respectively; the inhibitory activities against LAI.2 were higher than that of the T20 by 2762 folds and 9322 folds, respectively; and the inhibitory activities against 89.6 were higher than that of the T20 by 3311 folds and 5785 folds, respectively.

### Example 6 Broad-Spectrum Anti-HIV Activity of Membrane Fusion Inhibitors of the Present Disclosure

HIV has a high variability, and it evolves into many subtypes and recombinant viruses, wherein subtype A, B and C of HIV-1 are the major viruses responsible for AIDS pandemic, while in China, the recombinant viruses A/E and B/C are the major ones. The present example further evaluated the broad-spectrum anti-HIV activities of the membrane fusion inhibitors of the present disclosure through a group of cell fusion experiments by utilizing pseudoviruses of 12 representative globally pandemic HIV-1 strains and , compared and analyzed the activities with those of T20.

### 1. Experimental Method

The used materials and methods were described in the literature published by the inventors' laboratory^{[7]}. The preparation method of the HIV-1 peusdoviruses and antiviral experiment were the same as the methods described in Example 2. The cell-cell fusion inhibition activity mediated by the HIV-1 envelope protein (Env) was detected by using a cell fusion experiment based on a DSP fluorescence reporting system.
(1) 293T cells (effector cells) were spread in a 96-well plate (at about 1.5×10⁴ cells/well), and target cells 293FT (about 1.5×10⁴ cells/mL) stably expressing CXCR4/CCR5 and DSP₈₋₁₁ were spread in a 10 cm cell culture dish, and then they were placed under the conditions of 37 °C and 5% CO₂ for culture.
(2) After 16-hour of culture, the HIV-1 Env expression plasmid and DSP₁₋₇ plasmid were co-transfected into the 293T effector cells at a 1:1 ratio.
(3) After 24-hour of transfection, a membrane fusion inhibitor was dissolved in deionized water and diluted to the initial concentration with a DMEM medium, and added to the effector cells (50 µL/well) in the 96-well plate in a 3-fold serial dilution, with 9 dilutions and 3 duplicate wells. The control group was treated with DMEM medium without the inhibitor. The plate was incubated at 37° C with 5% CO₂ for 1 hour.
(4) The 293FT cells were re-suspended to adjust the cell concentration to be about 30×10⁴ cells/mL, and EnduRen live cell substrate (Promega) was added thereto at the ratio of 1: 4000 and mixed well. The mixture was incubated under the conditions of 37 °C and 5% CO₂ for 30 minutes.
(5) The 293FT cells were added to HIV-1 effector cell wells at 100 µL/well. The plate was centrifuged for 1 minute at 300 g to make full contact between the effector cells and the target cells. After the mixture was cultured at 37°C for 1 hour, the luciferase activity (relative luminescence units, RLU) was detected, the inhibition rate curve was made, and the half-maximal inhibition concentration (IC₅₀) of the drug was calculated.

### 2. Experimental Results and Analysis

The inhibitory results of the membrane fusion inhibitors of the present disclosure against infections of representative pandemic HIV-1 strain pseudoviruses were shown in FIG. 6. T20 polypeptide inhibited 12 pseudoviruses with an average IC₅₀ value of 20052.09 pM, while the four lipopeptides LP-123, LP-125, LP-127 and LP-128 inhibited 12 pseudoviruses with average IC₅₀ values of 27.39 pM, 4.22 pM, 4.89 pM and 3.19 pM, respectively. By comparison, the inhibitory activities of the LP-123, LP-125, LP-127 and LP-128 against 12 pseudoviruses were higher than that of the T20 by 732 folds, 4752 folds, 4101 folds and 6286 folds, respectively.

The inhibitory results of the membrane fusion inhibitors of the present disclosure against the cell fusion mediated by representative pandemic HIV-1 strain Env were shown in FIG. 7. The T20 polypeptide inhibited the membrane fusions of 12 viruses with an average IC₅₀ value of 11214.53 pM, while the four lipopeptides LP-123, LP-125, LP-127 and LP-128 inhibited the membrane fusion of 12 viruses with average IC₅₀ values of 24.04 pM, 11.9 pM, 4.97 pM and 4.02 pM, respectively. By comparison, the inhibitory activities of the LP-123, LP-125, LP-127 and LP-128 against membrane fusions of 12 viruses were higher than that of the T20 by 466 folds, 942 folds, 2256 folds and 2790 folds, respectively.

### Example 7 Inhibitory Activity of Membrane Fusion Inhibitors of the Present Disclosure against T20-Resistant Virus Strains

Another important biological feature of HIV is that the HIV is prone to drug resistance, resulting in treatment failures, and this is a key problem to be solved in drug research and development. The present example evaluated the antiviral activities of the membrane fusion inhibitors of the present disclosure by using a group of highly T20-resistant mutant strains, and compared and analyzed their inhibitory activity with that of lipopeptide LP-98.

### 1. Experimental Method

Based on HIV-1 strain NL4-3, a group of twelve T20-resistant mutant strains (L33S, I37T, V38A, V38M, Q40H, N43K, L33S/I37T, G36S/V38M, I37T/N43K, V38A/N42T, L33S/V38A/N42T, N43K/E49A/N126K) with a single mutation, a double mutation or a triple mutation were constructed, and the construction method was described in the literatures published by the inventors' laboratory^{[7][11][12]}. The preparation method of the mutant strain pseudoviruses and antiviral experiment were the same as the methods described in Example 2.

### 2. Experimental Results and Analysis

The results were shown in FIG. 8. The results showed that the LP-98 inhibited 12 drug-resistant mutant strains with an average IC₅₀ value of 2663.27 pM, while the LP-123, LP-125, LP-127 and LP-128 inhibited 12 drug-resistant mutant strains with average IC₅₀ values of 55.51pM, 6.29 pM, 7.02 pM and 9.13 pM, respectively. By comparison, the inhibitory activities of the LP-123, LP-125, LP-127 and LP-128 against 12 drug-resistant mutant strains were higher than that of the LP-98 by 48 folds, 423 folds, 379 folds and 292 folds, respectively. Hence, the membrane fusion inhibitors of the present disclosure had potent inhibitory activities against T20-resistant virus strains, reflecting a remarkable advantage in the druggability.

### Example 8 Broad-Spectrum Inhibitory Activity of Membrane Fusion Inhibitors of the Present Disclosure against Other Viruses

The present example evaluated the inhibitory activities of LP-127 and LP-128 against SARS-CoV-2 and multiple other viruses by utilizing a pseudovirus system.

### 1. Experimental Method

The method for preparing pseudoviruses is a well-established technology in the field and can be used routinely for a person skilled in the art. Methods for the preparation of SARS-CoV-2 D614G and BA4/5 mutant strains, MERS-CoV, IAV (H7N9), and VSV pseudoviruses and antiviral experiment were described in the literatures published by the inventors' laboratory^{[13][14][15]}. The preparation of EBOV, MARV and RABV pseudoviruses was also described in the above literatures, which were obtained by co-transfecting a plasmid encoding the viral envelope proteins with the HIV-1 backbone plasmid pNL4-3.luc.R into HEK293T cells, and the steps of the antiviral experiment were substantially consistent with the method described in Example 2, with the main differences being the kind of the target cells and the amount of the pseudoviruses used. The target cell of SARS-CoV-2 pseudovirus was 293T/ACE2; the target cells of MERS-CoV, EBOV and MARV pseudoviruses was Huh-7; the target cell of H7N9 pseudovirus was MDCK; the target cell of RABV and VSV pseudoviruses was HEK293T. The amount of each pseudovirus used was 1000 TCID₅₀.

### 2. Experimental Results and Analysis

The results were shown in FIG. 9. It was surprisingly found by the inventors that lipopeptides LP-127 and LP-128 could effectively inhibit the infection of two SARS-CoV-2 mutant strains, wherein the LP-127 and the LP-128 inhibited D614G mutant strain with IC₅₀ values of 27.4 nM and 25.12 nM, respectively; and inhibited Omicron BA4/5 mutant strain with IC₅₀ values of 26.56 nM and 23.51 nM, respectively. The LP-127 and the LP-128 inhibited MERS-CoV with IC₅₀ value of 382.72 nM and 455.67 nM, respectively; inhibited EBOV with IC₅₀ value of 61.68 nM and 274.47 nM, respectively; inhibited MARV with IC₅₀ value of 143.98 nM and 534.44 nM, respectively; inhibited H7N9 with IC₅₀ value of 414.87 nM and 352.6 nM, respectively; inhibited RABV with IC₅₀ value of 1731 nM and 1130 nM, respectively; and inhibited VSV with IC₅₀ value of 1509 nM and 1490 nM, respectively.

The above experimental results showed that the LP-127 and the LP-128 not only could potently inhibit various HIV-1 subtypes and T20-resistant virus strains, but also could effectively inhibit multiple other viruses, and they were broad-spectrum antiviral candidate medicaments.

### Example 9 Analysis of the In Vitro Cytotoxicity of Lipopeptides LP-127 and LP-128

To clarify the antiviral activity specificity and druggability of the membrane fusion inhibitors LP-127 and LP-128 of the present disclosure, the present example further evaluated their in vitro cytotoxicitity.

### 1. Experimental Method

The in vitro cytotoxicity was detected by using a CCK-8 cell proliferation/toxicity assay kit (manufacturer: Abbkine, Cat. No.: KTC 011001). The specific steps were as follows: (1) in a 96-well plate, the test lipopeptide was diluted in a 3-fold gradient, with 9 dilutions and three duplicate wells for each dilution; and each well contained 100 µL of a lipopeptide solution; DMEM medium control wells (100 µL for each well) were set; (2) about 10×10⁴ cells/mL of test cells (TZM-bl, MT-4, MDCK, Huh-7, Hep-2, HEK293T, 293T/ACE2) suspension was added to the 96-well plate in step (1) at 100 µL/well, and cultured under the conditions of 37 °C and 5% CO₂ for 48 hours; (3) 20 µL of CCK-8 solution was added to each well and the plate continued to be incubated in an incubator for 2 hours; then the absorbance at 450 nm (OD 450) was measured with a microplate reader. By using GraphPad Prism software, the inhibition rate curve was fitted and the half-maximal cytotoxic concentration (CC₅₀) of the drug was calculated.

### 2. Experimental Results and Analysis

The results were shown in FIG. 10. The results showed that the LP-127 had an average CC₅₀ of 15.68 µM on seven test cells, wherein the CC₅₀ on TZM-bl cells was 10.21 µM, the CC₅₀ on MT-4 cells was 15.45 µM, the CC₅₀ on MDCK cells was 12.89 µM, the CC₅₀ on Huh-7 cells was 31.28 µM, the CC₅₀ on Hep-2 cells was 16.46 µM, the CC₅₀ on HEK293T cells was 9.07 µM, the CC₅₀ on 293T/ACE2 cells was 14.42 µM. The results showed that the LP-128 had an average CC₅₀ of 19.91 µM on seven test cells, wherein the CC₅₀ on TZM-bl cells was 7.49 µM, the CC₅₀ on MT-4 cells was 28.22 µM, the CC₅₀ on MDCK cells was 18.05 µM, the CC₅₀ on Huh-7 cells was 40.28 µM, the CC₅₀ on Hep-2 cells was 12.48 µM, the CC₅₀ on HEK293T cells was 8.72 µM, the CC₅₀ on 293T/ACE2 cells was 24.1 µM.

With CC₅₀/IC₅₀ analyses, it could be seen that both the LP-127 and the LP-128 had a very high therapeutic selective index (SI).

### Example 10 Stability Research of Lipopeptides LP-127 and LP-128

In order to further research the druggability of the membrane fusion inhibitors of the present disclosure, in the present example, the LP-127 and the LP-128 were placed at room temperature or 37 °C for a long time, incubated with human serum, or digested with protease, and then their stability was evaluated by detecting changes in their antiviral activity.

### 1. Experimental Method

Temperature Stability Experiment: LP-127 or LP-128 aqueous solutions at a concentration of 300 µM were placed at room temperature or 37 °C for various time periods and then their activities inhibiting NL4-3 pseudoviruses to infect TZM-bl cells were determined by the same method as that in Example 2.

Human Serum Stability Experiment: 20% human serum was mixed with LP-127 or LP-128 with a final concentration of 150 µm; the obtained mixture was incubated at 37 °C for 0, 5, 30, 60, 120, 180 or 240 minutes, and then their activities inhibiting NL4-3 pseudoviruses to infect TZM-bl cells were determined by the same method as that in Example 2.

Digestion with Protease: LP-127 or LP-128 was mixed with protease K, trypsin, or α-chymotrypsin (Sigma-Aldrich products, Cat. No.: P2308, T4799, and C4129, respectively) at a ratio of 20:1 (the final concentrations were 2 mg/mL and 0.1 mg/mL, respectively); the obtained mixtures were incubated at 37 °C for 0, 30, 60, 120, 180, or 240 minutes, respectively, and then their activities inhibiting NL4-3 pseudoviruses to infect TZM-bl cells were determined by the same method as that in Example 2.

### 2. Experimental Results and Analysis

The results were shown in FIG. 11. In comparison to the untreated lipopeptides (when the incubation time is 0), after treatment at different temperatures, with human serum, or with three proteases for various time periods, there was no significant change in the antiviral activity of LP-127 and LP-128, indicating that they were highly stable.

### Example 11 Helix Structural Characteristics of Membrane Fusion Inhibitors of the Present Disclosure and Binding Stability Analyses thereof

In order to analyze the structural features of the membrane fusion inhibitors of the present disclosure and investigate their mechanism of action, a circular dichroism (CD) technique was used to determine the secondary structure (α-helix) and thermal stability of LP-121, LP-122, LP-123, LP-124, LP-125, LP-126, LP-127 and LP-128 as well as their complexes with the target sequence N44.

### 1. Experimental Method

The CD determination method and the synthesis and preparation of the polypeptide N44 (Ac-TVQARQLLSGIVQQQNNLLRAIEAQQHLLQLTVWGIKQLQARIL-NH₂), which were derived from gp41 NHR sequence and served as an simulated target of inhibitor, were described in the literature published by the inventors^{[16]}.

Test lipopeptides and a mixture of N44 and lipopeptides were dissolved in a phosphate buffered saline (PBS) with a pH of 7.2, respectively, to obtain solutions having both the final concentrations of the lipopeptides and N44 polypeptide of 10 µM. Each solution was placed in a water bath at 37 °C for 30 minutes and then transferred to a corresponding cuvette. A JASCO spectropolarimeter (Model J-815) was used to scan changes of the molar ellipticity [θ]λ of the solution in the wavelength range of 195-270 nm. The typical α-helical structure could exhibit maximum negative peaks at 208 nm and 222 nm. The PBS blank control was subtracted to correct the spectrum values. During the calculations, the peak value of - 33000 degree.cm².dmol⁻¹ was use as the standard of 100% α-helix content, the percentages of the α-helix contents of the polypeptides were calculated according to the molar ellipticity of the solution at 222 nm. Subsequently, the solution was added to a corresponding cuvette for detecting thermal stability, and the CD temperature control module was adjusted to scan the polypeptide solution at a speed of 2 °C/min to detect the temperature-dependent changes in [θ]222 at the range of 20-98 °C. The melting curve was drawing and smoothed, and the midpoint temperature value (Tm) of the thermal dissociation transition was calculated using Origin software, to reflect the degree of helix thermal stability.

### 2. Experimental Results and Analysis

The results were shown in FIG. 12.

As shown in Figure 12A, the α-helix content of LP-121, LP-122, LP-123, LP-124, LP-125, LP-126, LP-127 or LP-128 alone was 69%, 65%, 56%, 64%, 78%, 51%, 57% or 66%, respectively, indicating that each lipopeptide inhibitor per se had a high helicity, wherein the LP-125 had the highest helicity. However, their Tm value could not be accurately calculated according to the melting curve.

As shown in Figure 12B, the α-helix content of the mixture of LP-121, LP-122, LP-123, LP-124, LP-125, LP-126, LP-127 or LP-128 and N44 was 81%, 81%, 79%, 79%, 89%, 66%, 68% or 84%, respectively, and their Tm values were >98 °C, >98 °C, >98 °C, 86 °C, 91 °C, 92 °C, 92 °C or 90 °C respectively, indicating that the lipopeptide inhibitors could interact with N44 to form a more stable α-helix structure, especially the three lipopeptides (LP-121, LP-122, LP-123) with a PBD containing amino acid W. The results also indicated that the substitution of W with Y could reduce the binding ability of the PBD, but the lipopeptides containing Y still had a relatively high binding stability (Tm >86 °C).

The present disclosure has been described in detail above. In the case of not departing from the spirit and scope of the present disclosure and in no need of conducting unnecessary experiments, one of ordinary skill in the art could implement the present disclosure in a broad scope at equivalent parameters, concentrations and conditions. While the present disclosure provides special examples, it will be appreciated that the present disclosure may be further modified. In general, according to the principles of the present disclosure, the present disclosure is intended to cover any alterations, uses, or improvements to the present disclosure, including changes made with known routine techniques in the art with departing from the scope as disclosed in the present disclosure. According to the scope of the claims attached below, some basic features may be applied.

### The References:

[1]. Chan DC, Kim PS. HIV entry and its inhibition. Cell 1998, 93: 681-684.
[2]. He Y. Synthesized peptide inhibitors of HIV-1 gp41-dependent membrane fusion. Curr Pharm Des 2013, 19: 1800-1809.
[3]. Dwyer JJ, Wilson KL, Davison DK, Freel SA, Seedorff JE, Wring SA, et al. Design of helical, oligomeric HIV-1 fusion inhibitor peptides with potent activity against enfuvirtide-resistant virus. Proc Natl Acad Sci U S A 2007, 104: 12772-12777.
[4]. He Y, Xiao Y, Song H, Liang Q, Ju D, Chen X, et al. Design and evaluation of sifuvirtide, a novel HIV-1 fusion inhibitor. J Biol Chem 2008, 283: 11126-11134.
[5]. Chong H, Yao X, Zhang C, Cai L, Cui S, Wang Y, et al. Biophysical property and broad anti-HIV activity of albuvirtide, a 3-maleimimidopropionic acid-modified peptide fusion inhibitor. PLoS One 2012, 7: e32599.
[6]. Ingallinella P, Bianchi E, Ladwa NA, Wang YJ, Hrin R, Veneziano M, et al. Addition of a cholesterol group to an HIV-1 peptide fusion inhibitor dramatically increases its antiviral potency. Proc Natl Acad Sci U S A 2009, 106: 5801-5806.
[7]. Xue J, Chong H, Zhu Y, Zhang J, Tong L, Lu J, et al. Efficient treatment and pre-exposure prophylaxis in rhesus macaques by an HIV fusion-inhibitory lipopeptide. Cell 2022, 185: 131-144 e118.
[8]. Yu D, Zhu Y, Jiao T, Wu T, Xiao X, Qin B, et al. Structure-based design and characterization of novel fusion-inhibitory lipopeptides against SARS-CoV-2 and emerging variants. Emerg Microbes Infect 2021, 10: 1227-1240.
[9]. Zhou J, Xu W, Liu Z, Wang C, Xia S, Lan Q, et al. A highly potent and stable pan-coronavirus fusion inhibitor as a candidate prophylactic and therapeutic for COVID-19 and other coronavirus diseases. Acta Pharm Sin B 2021.
[10]. Outlaw VK, Bovier FT, Mears MC, Cajimat MN, Zhu Y, Lin MJ, et al. Inhibition of Coronavirus Entry In Vitro and Ex Vivo by a Lipid-Conjugated Peptide Derived from the SARS-CoV-2 Spike Glycoprotein HRC Domain. mBio 2020,11:e01935-01920.
[11]. 11. Hu Y, Yu W, Geng X, Zhu Y, Chong H, He Y. In Vitro Selection and Characterization of HIV-1 Variants with Increased Resistance to LP-40, Enfuvirtide-Based Lipopeptide Inhibitor. Int J Mol Sci 2022, 23.
[12]. Wu X, Liu Z, Ding X, Yu D, Wei H, Qin B, et al. Mechanism of HIV-1 Resistance to an Electronically Constrained alpha-Helical Peptide Membrane Fusion Inhibitor. J Virol 2018, 92: e02044-02017.
[13]. Yu D, Zhu Y, Yan H, Wu T, Chong H, He Y. Pan-coronavirus fusion inhibitors possess potent inhibitory activity against HIV-1, HIV-2, and simian immunodeficiency virus. Emerg Microbes Infect 2021, 10: 810-821.
[14]. Zhu Y, Hu Y, Liu N, Chong H, He Y. Potent inhibition of diverse Omicron sublineages by SARS-CoV-2 fusion-inhibitory lipopeptides. Antiviral Res 2022, 208: 105445.
[15]. Zhu Y, Yu D, Hu Y, Wu T, Chong H, He Y. SARS-CoV-2-derived fusion inhibitor lipopeptides exhibit highly potent and broad-spectrum activity against divergent human coronaviruses. Signal Transduct Target Ther 2021, 6: 294.
[16]. Zhu Y, Ding X, Yu D, Chong H, He Y. The Tryptophan-Rich Motif of HIV-1 gp41 Can Interact with the N-Terminal Deep Pocket Site: New Insights into the Structure and Function of gp41 and Its Inhibitors. J Virol 2019, 94: effff1234.

## Claims

1. A lipopeptide or a pharmaceutically acceptable salt, a solvate, a hydrate, a complex, a chelate, a non-covalent complex or a prodrug thereof, comprising a polypeptide or a variant thereof and a modification group linked to the C-terminus of the polypeptide or the variant thereof through a linker, and optionally a terminal protecting group of the polypeptide,
(X₁X₂X₃)mX₄X₅X₆X₇X₈X₉X₁₀X₁₁X₁₂X₁₃X₁₄X₁₅IX₁₆X₁₇LX₁₈X₁₉X₂₀X₂₁X₂₂X₂₃QQX₂₄X₂₅N(EX₂₆)n Formula I
wherein, the polypeptide is represented by Formula I, the modification group is a lipophilic compound, the linker is -(EAAAK)n₁-, -(XP)n₂-, -(EAAAK)n₁-X₂₇- or -(XP)n₂-X₂₇-;
X₁ is T, E, or S;
X₂ is W or a hydrophilic amino acid;
X₃ is E, M or Q;
m is 0 or 1;
X₄ is E, A or T;
X₅ is W or a hydrophilic amino acid;
X₆ is E or D;
X₇ is R, K or Q;
X₈ is E, K or A;
X₉ is L or I;
X₁₀ is E, N or A;
X₁₁ is E or N;
X₁₂ is L or Y;
X₁₃ is E, T or A;
X₁₄ is K, S, R or A;
X₁₅ is K, L, R or Q;
X₁₆ is E, H, T or Y;
X₁₇ is E, S, R or A;
X₁₈ is L or I;
X₁₉ is K, E or R;
X₂₀ is K, E, Q or A;
X₂₁ is A or S;
X₂₂ is E or Q;
X₂₃ is E, N or I;
X₂₄ is K, E or D;
X₂₅ is K or R;
X₂₆ is Q, E, R, A or Y;
n is 0 or 1;
X is any one amino acid;
X₂₇ is K or C;
n₁ is a natural number between 1 and 5;
n₂ is a natural number between 1 and 5;
and the variant differs from the polypeptide from which the variant is derived only in the substitution (e.g., a conservative substitution or non-conservative substitution) of one or several (such as 1, 2, 3, 4 or 5) amino acid residues, and retains the biological function of the polypeptide from which the variant is derived.

2. The lipopeptide or a pharmaceutically acceptable salt, a solvate, a hydrate, a complex, a chelate, a non-covalent complex or a prodrug thereof according to claim 1, **characterized in** one or more of the following characteristics:
- X₁ is T or E;
- X₂ is W, D, E, H, K, Q, R, S, T or Y;
- X₃ is E or M;
- X₄ is E;
- X₅ is W, D, E, H, K, Q, R, S, T or Y;
- X₆ is E;
- X₇ is R or K;
- X₈ is E or K;
- X₁₀ is E or N;
- X₁₁ is E;
- X₁₂ is L;
- X₁₃ is E or T;
- X₁₄ is K or S;
- X₁₅ is K or L;
- X₁₆ is E or H;
- X₁₇ is E or S;
- X₁₈ is L;
- X₁₉ is K or E;
- X₂₀ is K or E;
- X₂₁ is A;
- X₂₂ is E;
- X₂₃ is E or N;
- X₂₄ is K or E;
- X₂₅ is K;
- X₂₆ is Q;
- X is A, K or E.

3. The lipopeptide or a pharmaceutically acceptable salt, a solvate, a hydrate, a complex, a chelate, a non-covalent complex or a prodrug thereof according to claim 1, **characterized in** one or more of the following characteristics:
- X₂ is W or Y, preferably Y;
- X₃ is E;
- X₅ is W or Y, preferably Y;
- X₇ is K;
- X₁₀ is E;
- X₁₃ is E;
- X₁₄ is K;
- X₁₅ is K;
- X₁₆ is E;
- X₁₇ is E;
- X₁₉ is K;
- X₂₀ is K;
- X₂₃ is E;
- X₂₄ is K.

4. The lipopeptide or a pharmaceutically acceptable salt, a solvate, a hydrate, a complex, a chelate, a non-covalent complex or a prodrug thereof according to any one of claims 1 to 3, wherein the polypeptide is represented by Formula I, Formula III, Formula IV or Formula V,
(X₁X₂X₃)mX₄X₅X₆X₇X₈X₉X₁₀X₁₁X₁₂X₁₃X₁₄X₁₅IX₁₆X₁₇LX₁₈X₁₉X₂₀X₂₁X₂₂X₂₃QQX₂₄KN(EX₂₆)n Formula II
(X₁X₂X₃)m-EX₅X₆X₇X₈X₉X₁₀X₁₁X₁₂X₁₃X₁₄X₁₅IX₁₆X₁₇LX₁₈X₁₉X₂₀X₂₁X₂₂X₂₃QQX₂₄KN(EX₂₆)n Formula III
(X₁X₂X₃)m-EX₅X₆X₇X₈X₉EELEKKIEELLKKAEEQQKKN(EX₂₆)n Formula IV
(X₁X₂X₃)m-EX₅EKX₈X₉EELEKKIEELLKKAEEQQKKN(EX₂₆)n Formula V
in which the definitions of X₁, X₂, X₃, m, X₄, X₅, X₆, X₇, X₈, X₉, X₁₀, X₁₁, X₁₂, X₁₃, X₁₄, X₁₅, X₁₆, X₁₇, X₁₈, X₁₉, X₂₀, X₂₁, X₂₂, X₂₃, X₂₄, X₂₆, and n are as described in any one of claims 1 to 3.

5. The lipopeptide or a pharmaceutically acceptable salt, a solvate, a hydrate, a complex, a chelate, a non-covalent complex or a prodrug thereof according to any one of claims 1 to 3, wherein the lipophilic compound is cholesterol (such as cholesteryl hemisuccinate, 2-cholesteryl acetic acid, 2-cholesteryl propionic acid, 3-cholesteryl propionic acid, 2-cholesteryl butyric acid, 2-cholesteryl isobutyric acid, 3-cholesteryl butyric acid, 3-cholesteryl isobutyric acid, 4-cholesteryl butyric acid, 2-cholesteryl valeric acid, 2-cholesteryl isovaleric acid, 3-cholesteryl valeric acid, 5-cholesteryl valeric acid, 2-cholesteryl caproic acid, 6-cholesteryl caproic acid, 2-cholesteryl enanthic acid, 7-cholesteryl enanthic acid, 2-cholesteryl caprylic acid, 8-cholesteryl caprylic acid, and cholesteryl bromoacetate), fatty acid (e.g., a fatty acid containing 8 to 20 carbon atoms), dihydrosphingosine or Vitamin E.

6. The lipopeptide or a pharmaceutically acceptable salt, a solvate, a hydrate, a complex, a chelate, a non-covalent complex or a prodrug thereof according to claim 5, wherein the fatty acid containing 8 to 20 carbon atoms is palmitic acid or octadecanoic acid.

7. The lipopeptide or a pharmaceutically acceptable salt according to any one of claims 1 to 3, wherein the lipopeptide has an amino acid sequence selected from amino acid sequences as set forth in SEQ ID NO: 11-19.

8. A polypeptide or a variant thereof, comprising or consisting of a sequence represented by Formula I:
(X₁X₂X₃)mX₄X₅X₆X₇X₈X₉X₁₀X₁₁X₁₂X₁₃X₁₄X₁₅IX₁₆X₁₇LX₁₈X₁₉X₂₀X₂₁X₂₂X₂₃QQX₂₄X₂₅N(EX₂₆)n Formula I
wherein
X₁ is T, E, S;
X₂ is W or a hydrophilic amino acid;
X₃ is E, M or Q;
m is 0 or 1;
X₄ is E, A or T;
X₅ is W or a hydrophilic amino acid;
X₆ is E or D;
X₇ is R, K or Q;
X₈ is E, K or A;
X₉ is L or I;
X₁₀ is E, N or A;
X₁₁ is E or N;
X₁₂ is L or Y;
X₁₃ is E, T or A;
X₁₄ is K, S, R or A;
X₁₅ is K, L, R or Q;
X₁₆ is E, H, T or Y;
X₁₇ is E, S, R or A;
X₁₈ is L or I;
X₁₉ is K, E or R;
X₂₀ is K, E, Q or A;
X₂₁ is A or S;
X₂₂ is E or Q;
X₂₃ is E, N or I;
X₂₄ is K, E or D;
X₂₅ is K or R;
X₂₆ is Q, E, R, A or Y;
n is 0 or 1;
and the variant differs from the polypeptide from which the variant is derived only in the substitution (e.g., a conservative substitution or non-conservative substitution) of one or several (such as 1, 2, 3, 4 or 5) amino acid residues, and retains the biological function of the polypeptide from which the variant is derived.

9. The polypeptide or the variant thereof according to claim 8, **characterized in** one or more of the following characteristics:
- X₁ is T or E;
- X₂ is W, D, E, H, K, Q, R, S, T or Y;
- X₃ is E or M;
- X₄ is E;
- X₅ is W, D, E, H, K, Q, R, S, T or Y;
- X₆ is E;
- X₇ is R or K;
- X₈ is E or K;
- X₁₀ is E or N;
- X₁₁ is E;
- X₁₂ is L;
- X₁₃ is E or T;
- X₁₄ is K or S;
- X₁₅ is K or L;
- X₁₆ is E or H;
- X₁₇ is E or S;
- X₁₈ is L;
- X₁₉ is K or E;
- X₂₀ is K or E;
- X₂₁ is A;
- X₂₂ is E;
- X₂₃ is E or N;
- X₂₄ is K or E;
- X₂₅ is K;
- X₂₆ is Q.

10. The polypeptide or the variant thereof according to claim 8, **characterized in** one or more of the following characteristics:
- X₂ is W or Y, preferably Y;
- X₃ is E;
- X₅ is W or Y, preferably Y;
- X₇ is K;
- X₈ is E or K;
- X₁₀ is E;
- X₁₃ is E;
- X₁₄ is K;
- X₁₅ is K:
- X₁₆ is E;
- X₁₇ is E;
- X₁₉ is K;
- X₂₀ is K;
- X₂₃ is E;
- X₂₄ is K.

11. The polypeptide or the variant thereof according to any one of claims 8 to 10, wherein the polypeptide is represented by Formula II, Formula III, Formula IV or Formula V,
(X₁X₂X₃)mX₄X₅X₆X₇X₈X₉X₁₀X₁₁X₁₂X₁₃X₁₄X₁₅IX₁₆X₁₇LX₁₈X₁₉X₂₀X₂₁X₂₂X₂₃QQX₂₄KN(EX₂₆)n Formula II
(X₁X₂X₃)m-EX₅X₆X₇X₈X₉X₁₀X₁₁X₁₂X₁₃X₁₄X₁₅IX₁₆X₁₇LX₁₈X₁₉X₂₀X₂₁X₂₂X₂₃QQX₂₄KN(EX₂₆)n Formula III
(X₁X₂X₃)m-EX₅X₆X₇X₈X₉EELEKKIEELLKKAEEQQKKN(EX₂₆)n Formula IV
(X₁X₂X₃)m-EX₅EKX₈X₉EELEKKIEELLKKAEEQQKKN(EX₂₆)n Formula V
in which the definitions of X₁, X₂, X₃, m, X₄, X₅, X₆, X₇, X₈, X₉, X₁₀, X₁₁, X₁₂, X₁₃, X₁₄, X₁₅, X₁₆, X₁₇, X₁₈, X₁₉, X₂₀, X₂₁, X₂₂, X₂₃, X₂₄, X₂₆, and n are as described in any one of claims 8 to 10.

12. The polypeptide or the variant thereof according to any one of claims 8 to 10, wherein the lipopeptide has an amino acid sequence selected from amino acid sequences as set forth in SEQ ID NO: 21-29.

13. A conjugate comprising the polypeptide or the variant thereof according to any one of claims 8 to 12 and a modification group;
for example, the modification group is linked to the N-terminus or C-terminus of the polypeptide or the variant thereof, optionally through a linker;
for example, the modification group is a terminal protecting group.

14. An isolated nucleic acid, encoding the polypeptide or the variant thereof according to any one of claims 8 to 12.

15. A vector comprising the isolated nucleic acid according to claim 14.

16. A host cell comprising the isolated nucleic acid according to claim 14 and/or the vector according to claim 15.

17. A multimer formed with the lipopeptide or a pharmaceutically acceptable salt, a solvate, a hydrate, a complex, a chelate, a non-covalent complex or a prodrug thereof according to any one of claims 1 to 7 or the polypeptide or the variant thereof according to any one of claims 8 to 10.

18. A composition, comprising the lipopeptide or a pharmaceutically acceptable salt, a solvate, a hydrate, a complex, a chelate, a non-covalent complex or a prodrug thereof according to any one of claims 1 to 7, or the polypeptide or the variant thereof according to any one of claims 8 to 12, or the conjugate according to claim 13, or the isolated nucleic acid according to claim 14, or the vector according to claim 15, or the host cell according to claim 16, or the multimer according to claim 17.

19. A pharmaceutical composition, comprising the lipopeptide or a pharmaceutically acceptable salt, a solvate, a hydrate, a complex, a chelate, a non-covalent complex or a prodrug thereof according to any one of claims 1 to 7, or the polypeptide or the variant thereof according to any one of claims 8 to 10, optionally further comprising a pharmaceutically acceptable carrier and/or excipient;
preferably, the lipopeptide or a pharmaceutically acceptable salt, a solvate, a hydrate, a complex, a chelate, a non-covalent complex or a prodrug thereof or the polypeptide or the variant thereof is present in an amount effective for treating a viral infection or a disease caused by viral infection;
preferably, the viral infection is an infection caused by a virus selected from the group consisting of HIV and a drug-resistant strain thereof, SARS-CoV-2 and a mutant strain thereof, MERS-CoV, EBOV, MARV, IAV, RABV and VSV;
preferably, the viral infection is an infection caused by HIV or a drug-resistant strain thereof.

20. A method for inhibiting viral membrane infusion or a method for treating a viral infection or a disease caused by viral infection, comprising administering an effective amount of the lipopeptide or a pharmaceutical acceptable salt, a solvate, a hydrate, a complex, a chelate, a non-covalent complex or a prodrug thereof according to any one of claims 1 to 7 or the polypeptide or the variant thereof according to any one of claims 8 to 10 or the pharmaceutical composition according to claim 19 to a subject in need thereof;
preferably, the viral infection is an infection caused by a virus selected from the group consisting of HIV and a drug-resistant strain thereof, SARS-CoV-2 and a mutant strain thereof, MERS-CoV, EBOV, MARV, IAV, RABV and VSV;
preferably, the viral infection is an infection caused by HIV or a drug-resistant strain thereof.

21. Use of the lipopeptide or a pharmaceutical acceptable salt, a solvate, a hydrate, a complex, a chelate, a non-covalent complex or a prodrug thereof according to any one of claims 1 to 7 or the polypeptide or the variant thereof according to any one of claims 8 to 10 in the manufacture of a pharmaceutical composition, wherein the pharmaceutical composition is used as a viral membrane fusion inhibitor or for treating a viral infection or a disease caused by viral infection;
preferably, the viral infection is an infection caused by a virus selected from the group consisting of HIV and a drug-resistant strain thereof, SARS-CoV-2 and a mutant strain thereof, MERS-CoV, EBOV, MARV, IAV, RABV and VSV;
preferably, the viral infection is an infection caused by HIV or a drug-resistant strain thereof.

22. The lipopeptide or a pharmaceutical acceptable salt, a solvate, a hydrate, a complex, a chelate, a non-covalent complex or a prodrug thereof according to any one of claims 1 to 7 or the polypeptide or the variant thereof according to any one of claims 8 to 10, for use as a viral membrane fusion inhibitor or for use in treating a viral infection or a disease caused by viral infection;
preferably, the viral infection is an infection caused by a virus selected from the group consisting of HIV and a drug-resistant strain thereof, SARS-CoV-2 and a mutant strain thereof, MERS-CoV, EBOV, MARV, IAV, RABV and VSV;
preferably, the viral infection is an infection caused by HIV or a drug-resistant strain thereof.

23. A pharmaceutical composition for inhibiting viral membrane fusion or a pharmaceutical composition for treating a viral infection or a disease caused by viral infection, which comprises the lipopeptide or a pharmaceutical acceptable salt, a solvate, a hydrate, a complex, a chelate, a non-covalent complex or a prodrug thereof according to any one of claims 1 to 7 or the polypeptide or the variant thereof according to any one of claims 8 to 10;
preferably, the viral infection is an infection caused by a virus selected from the group consisting of HIV and a drug-resistant strain thereof, SARS-CoV-2 and a mutant strain thereof, MERS-CoV, EBOV, MARV, IAV, RABV and VSV;
preferably, the viral infection is an infection caused by HIV or a drug-resistant strain thereof.
